(19) Europäisches Patentamt
European Patent Office
Office européen des brevets

(11) **EP 4 692 300 A1**

(12) **EUROPEAN PATENT APPLICATION**
published in accordance with Art. 153(4) EPC

(43) Date of publication:
**11.02.2026 Bulletin 2026/07**

(21) Application number: **24777929.1**

(22) Date of filing: **22.03.2024**

(51) International Patent Classification (IPC):
*C12M 1/34* (2006.01)     *C12M 1/00* (2006.01)
*C12Q 1/02* (2006.01)     *G06V 20/69* (2022.01)

(52) Cooperative Patent Classification (CPC):
**C12M 1/00; C12M 1/34; C12M 1/42; C12Q 1/02;**
**G01N 11/00; G01N 21/55; G01N 33/543;**
**G06V 20/69**

(86) International application number:
**PCT/CN2024/083361**

(87) International publication number:
**WO 2024/199140 (03.10.2024 Gazette 2024/40)**

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**GE KH MA MD TN**

(30) Priority: **24.03.2023   CN 202310299296**
**24.03.2023   CN 202310299292**
**24.03.2023   CN 202310299290**
**24.03.2023   CN 202310299306**
**24.03.2023   CN 202310299309**
**24.03.2023   CN 202310297791**
**24.03.2023   CN 202310299308**

(71) Applicant: **Yigong Ruixin (Xiamen) Technology**
**Co., Ltd**
**Xiamen, Fujian 361015 (CN)**

(72) Inventor: **LIN, Zhe**
**Xiamen, Fujian 361015 (CN)**

(74) Representative: **Chung, Hoi Kan**
**Mandarin IP Limited**
**7 Cherry Trees**
**Great Shelford**
**Cambridge CB22 5XA (GB)**

(54) **DRUG SENSITIVITY-BASED CELL CHARACTERIZATION AND IDENTIFICATION TYPING SYSTEM, METHOD AND USE**

(57)     This application relates to the field of biotechnology, and more particularly to a system, method, and application for cell identification, classification, and characterization based on drug sensitivity.

The cell characterization system comprises a cell mechanical force detection device. The cell mechanical force detection device includes a base and, disposed on the base, a micropillar array composed of at least one micropillar that can deform under the cellular mechanical force exerted by cells or multicellular aggregates. The tops and/or upper surfaces of the micropillars are provided with a light-reflective layer. The cells or multicellular aggregates exhibit a corresponding degree of drug sensitivity to specific drugs.

The cell identification and classification system includes an information acquisition unit, a pre-processing unit, a learning unit, and a recognition unit. This application distinguishes between cells or multicellular aggregates with different degrees of drug resistance by using cellular mechanical forces. This identification can be completed in a short period, thereby enabling the screening of precision medicines with an accuracy rate of over 98%.

Figure 7

**Description**

**TECHNICAL FIELD**

This application relates to the field of biotechnology, and more particularly to a system, method, and application for the characterization and identification/classification of cells based on drug sensitivity.

**BACKGROUND ART**

The sensitivity of cells in the human body to drugs can be categorized into cells with different degrees of drug resistance and non-drug-resistant cells, which can be further divided into drug-resistant tumor cells and non-drug-resistant tumor cells.

Drug resistance, also known as chemoresistance, refers to the tolerance of microorganisms, parasites, and tumor cells to the effects of drugs. Once drug resistance develops, the efficacy of the drug is significantly reduced. Drug resistance can be classified into acquired resistance and natural resistance based on its origin. A particular strain of a pathogen in nature, such as bacteria, may also exhibit natural resistance. With the long-term use of antibiotics, the majority of sensitive strains are continuously killed, allowing resistant strains to proliferate extensively and replace the sensitive ones, leading to a continuous increase in the drug resistance rate of the bacteria to that specific drug. Currently, methods for evaluating cell drug resistance rely on animal testing or cell culture, which are time-consuming and costly, making it unsuitable for patients in urgent need of drug treatment and difficult to discover and screen for primary drug-resistant cells. In cancer patients, drugs that are effective against tumors often have a cytotoxic effect on normal cells, causing severe or even devastating damage to other organs of the patient's body while treating the cancer. Therefore, traditional systems and methods for the characterization and identification/classification of drug-sensitive cells suffer from defects such as insufficient sensitivity, limited specificity, being time-consuming and operationally cumbersome, strong subjectivity, high cost, and the inability to comprehensively reflect cellular characteristics.

**SUMMARY OF THE INVENTION**

**(I) Technical Problem to be Solved**

In view of the above-mentioned shortcomings and deficiencies of the prior art, this application provides a characterization system and method for cells based on drug sensitivity, which can acquire subtle changes in biological and physical information such as cell mechanical force, and can be used to characterize the types and states of cells and multicellular aggregates in a static state, during the culture process, under external stimulation, and after external stimulation.

Correspondingly, this application also provides a system for the characterization and identification/classification of cells based on drug sensitivity, which can identify cell types such as cells with different degrees of drug resistance in a short period of time, at low cost, and with high throughput, thereby enabling the screening of precision medicines.

Correspondingly, this application also provides the application of the characterization system and identification/classification system for cells based on drug sensitivity in methods including the detection of interactions between cells and multicellular aggregates, for the precision screening of drugs in different methods, which can screen for drugs with different degrees of resistance, and drugs that are effective against tumors but non-invasive to normal cells, among others.

**(II) Technical Solution**

To achieve the above objectives, the main technical solutions employed in this application include:
In first aspect, this application provides a method for identifying cell drug resistance level, comprising:

acquiring cell physical information, wherein the cell physical information comprises cell mechanical force and/or cell stiffness;

identifying cells with different drug resistance level, as well as non-drug-resistant cells, based on the cell physical information.

In this application, "degree of cell drug resistance" or drug resistance level refers to the corresponding degree of drug sensitivity exhibited by cells or multicellular aggregates to a specific drug. Therefore, in this type of expression, the term "cell" should be broadly understood as a single cell or a cell population (multicellular aggregate) composed of two or more cells. In the definition of this application, a multicellular aggregate refers to: a cell being the basic structural and functional unit of an organism, and cells usually proliferating or differentiating to form two or more cells clustered together to form a cell population. Multicellular aggregates include cell groups obtained from in vitro or in vivo culture, such as tumor aggregates, including organoids and active tissues. The cells with different degrees of drug resistance referred to in this application refer to cells that have different levels of resistance to a certain drug, such as the multiple subtypes of tumor cells classified due to their different degrees of drug resistance.

In an optional embodiment of this application, the cell mechanical force comprises one or more of the magnitude, direction, distribution, and frequency of the cell mechanical force, and preferably comprises the change over time of one or more of the magnitude, direction, distribution, and frequency of the cell mechanical force;

said cell stiffness comprises the magnitude of cell stiffness at specific locations within the cell, and preferably comprises the magnitude of stiffness of different layers at specific locations within the cell, their spatial distribution, and their change over time;

preferably, said cell physical information is obtained before, during, and/or after the action of a specific drug;

preferably, said cell physical information comprises the cell mechanical force and/or cell stiffness acquired during at least one of the following: interactions between cells and multicellular aggregates, interactions between cells, interactions between multicellular aggregates, the effect of substances on cells and/or multicellular aggregates, and the effect of other physical, biological, or chemical factors on cells and/or multicellular aggregates.

In some optional embodiments, said cell physical information comprises a combination of one or more of cell number, cell mechanical force, cell stiffness, and cell morphology.

In some optional embodiments, the method for identifying the degree of cell drug resistance further comprises the step: quantifying the degree of drug resistance of said cells with different degrees of drug resistance. In this way, not only can drug-resistant and non-drug-resistant cells be distinguished, but the drug-resistant cells can also be further classified into different resistance levels.

In some optional embodiments, the method for identifying the degree of cell drug resistance further comprises: applying stimuli of different types and intensities during the acquisition of the cell physical information,

optionally, the stimuli are a combination of one or more of physical stimuli, chemical stimuli, and biological stimuli;

optionally, the physical, chemical, and biological stimuli comprise is selected from a group consisted of drugs, mechanical force, stiffness, biochemicals, electric fields, flow fields, tropic guidance, and radiation. Among them, tropic guidance is: guiding the growth and change of cells in the direction of scratches by making scratches on the substrate in a certain direction. In an optional solution, when the external stimulus is a specific microenvironment, it can amplify the differences in cell mechanical force information between different subtypes of cells, making them easier to identify and improving the efficiency and accuracy of identification. For example, if it is difficult to accurately distinguish the differences between two different subtypes of cells in a softer environment, applying a stiffness stimulus through a harder environment can amplify the differences between the cell subtypes, thereby enabling more accurate identification.

In some other embodiments, the method for identifying cell drug resistance level further comprises: comparing and analyzing the result identified based on the cell physical information with results from biochemical and/or optical characterization, wherein the biochemical and/or optical characterization comprises at least one of protein staining, immunohistochemical staining imaging characterization, and single-cell sequencing.

As a preferred embodiment of this application, the method for identifying cell drug resistance level comprises performing characterization using a cell characterization system, wherein the cell characterization system comprises: a cell mechanical force detection device for acquiring cell mechanical forcethe cell mechanical force detection device comprises: a base, and disposed on the base, a micropillar array composed of one or more micropillars that can deform under the cellular mechanical force of the cells,
the micropillars comprise a bottom end connected to the base, a side surface, a pillar body enclosed by the side surface, and a top end opposite the bottom end and away from the base, wherein the micropillars have a light-reflective layer; optionally, the base and the pillar body of the micropillars have a light-transmissive portion, and the top end of the micropillars has a light-reflective layer.

Specifically, the multicellular aggregates of this application are attached to the cell mechanical force detection device in various ways, two of which are as follows:

First attachment method: A culture medium is provided on the micropillars of the cell mechanical force detection device, and cells are transplanted into the culture medium on the micropillars to be cultured into multicellular aggregates. In other embodiments, this attachment method allows for real-time monitoring of the cell culture process when the cell mechanical force information is output in a visual format, which can be applied to study the effects of chemical, biological, and physical external stimuli, such as culture media and drugs, on cell growth.

Second attachment method: The pre-cultured multicellular aggregates are directly adhered to the micropillars of the cell mechanical force detection device for detection.

the optical signal generation device has a light source, and the light emitted from the light source illuminates the light-reflective layer via an incident light path;

the optical signal detection device is used to detect the light reflected from the light-reflective layer, wherein the light reflected from the light-reflective layer enters the optical signal detection device via a reflected light path;

optionally, the light intensity acquired by the optical signal detection device is linearly correlated with the cell mechanical force within a certain range.

The cell characterization system further comprises: an optical signal analysis device, which transforms the optical signal analysis results into visual information or data;
optionally, the optical signal analysis device is used to calculate the cell mechanical force of each micropillar based on the attenuation of the reflected light intensity, and to determine the type, state, and behavior of the detected cells according to a preset model.

Second aspect, this application provides a system for identification and classification of cells based on drug sensitivity, which comprises: an information acquisition unit and a pre-processing unit,

the information acquisition unit is for acquiring cell physical information, wherein the cell physical information comprises cell mechanical force and/or cell stiffness;

the pre-processing unit is for pre-processing the cell physical information to form structured cell information.

Preferably, the cell mechanical force comprises at least one of the magnitude, direction, distribution, and frequency of the cell mechanical force, and preferably comprises the change over time of at least one of the magnitude, direction, distribution, and frequency of the cell mechanical force;

the cell stiffness comprises the magnitude of cell stiffness at specific locations within the cell, and preferably comprises the magnitude of stiffness of different layers at specific locations within the cell, their spatial distribution, and their change over time;

preferably, the cell physical information is obtained before, during, and/or after the action of a specific drug.

In a still further preferred embodiment, the cell physical information comprises a combination of at least one of cell number, cell mechanical force, cell stiffness, and cell morphology.

In some preferred embodiments, the cell identification and classification system further comprises a learning unit and a recognition unit,

the learning unit is for using the structured cell information as input data to establish a cell feature model using supervised, unsupervised, or semi-supervised machine learning;

the recognition unit is for applying the cell feature model to the classification or clustering of cells based on drug sensitivity, to achieve the identification of cell types based on drug sensitivity.

In second aspect, the inventor provides a method for acquiring cell physical information based on drug sensitivity, wherein the cell physical information comprises cell mechanical force and/or cell stiffness, comprising the following steps:

obtaining a number of original cells and acquiring the cell physical information of each original cell;

performing specific drug screening on the original cells to obtain cells with different drug resistance level;

acquiring the cell physical information of the cells with different drug resistance level;

comparing the cell mechanical force information of the cells with different drug resistance level with the cell mechanical force information of the corresponding original cells, wherein cells with a reduction in cell mechanical force less than a first preset threshold during and/or after drug treatment are drug-resistant cells, and cells with a reduction greater than or equal to a second preset threshold are non-drug-resistant cells; or

comparing the cell stiffness of the cells with different drug resistance level with the cell stiffness of the corresponding original cells, wherein cells with a reduction in cell stiffness less than a third preset threshold during and/or after drug treatment are drug-resistant cells, and cells with a reduction greater than or equal to a fourth preset threshold are non-drug-resistant cells;

acquiring cell physical information of the drug-resistant and non-drug-resistant cells for the specific drug.

In third aspect, the inventor provides a method for identification and classification of cells based on drug sensitivity, wherein the identification and classification are performed by acquiring cell physical information of the cells; the cell physical information comprises at least one of cell mechanical force information;

optionally, cell mechanical force informationcomprises at least one of cell mechanical force information acquired before the action of external factors, during the action of external factors, and after the action of external factors;

optionally, classifying cell types according to different degrees of drug resistance;

optionally, the cell types include: normal cells and pathological cells, activated cells and non-activated cells;

optionally, the cell mechanical force of drug-resistant cells with EGFR mutations is large and uniformly dispersed throughout the entire cell, while the cell mechanical force of non-drug-resistant cells is small and concentrated in the peripheral regions of the cell.

In some improved embodiments of the above method for identification and classification of cells based on drug sensitivity, the following steps are included:

acquiring cell physical information of cells based on drug sensitivity, wherein the cell physical information includes cell mechanical force information and cell stiffness information at a certain point in the cell, the cell mechanical force information comprises the magnitude, direction, frequency, distribution, and dynamic change over time of the cell mechanical force at specific locations of the cell or multicellular aggregate, and the cell stiffness information comprises the magnitude of stiffness of different layers at specific locations within the cell, their spatial distribution, and their change over time;

pre-processing the cell physical information to form structured cell information; the structured cell information comprises the number of cells, the number of cell features, and the feature information of each cell feature;

using the structured cell information as input data to establish a cell feature model using supervised, unsupervised, or semi-supervised machine learning, and applying the cell feature model to the classification or clustering of cells based on drug sensitivity of unknown type or unknown state.

, the inventor provides an application of the identification method as described in the first aspect of this application, the system for identification and classification of cells based on drug sensitivity as described in the second aspect of this application, the method for acquiring cell mechanical force of cells and/or multicellular aggregates based on drug sensitivity as described in the third aspect of this application, and the method for identification and classification of cells based on drug sensitivity as described in the fourth aspect of this application, wherein the application comprises: precision drug screening, screening of drug-resistant cells, and drug discovery.

Optionally, the above application comprises:

classifying cells and/or multicellular aggregates by acquiring the cell mechanical force of two or more cells before, during, and after drug action, to predict the effectiveness of the drug and achieve precision screening of therapeutic drugs; or

classifying a single cell's response to different drugs by acquiring its state before, during, and after the action of different drugs, to screen for the most effective drug or drug combination and achieve precision screening of therapeutic drugs.

Optionally, applying physical, biological, and/or chemical stimuli to the cells to be tested, and respectively acquiring the cell mechanical force information and/or its dynamic changes under different conditions and before and after applying the stimuli; identifying the stimuli and micro-environmental conditions that can amplify the differences between different cell types;

Optionally, acquiring the cell mechanical force information of tumor cells and normal cells, respectively, under the action of different therapeutic drugs; identifying therapeutic drugs and their concentrations that have no killing effect or little impact on normal cells but are effective against tumor cells.

**(III) Beneficial Effects**

In contrast to the prior art, the beneficial effects of this application are:

First, this application distinguishes between cells or multicellular aggregates with different degrees of drug resistance through cell mechanical force, which can identify whether they are drug-resistant or non-drug-resistant to different drugs in a short period of time, at low cost, and with high throughput, thereby enabling the screening of precision medicines with an accuracy rate of over 98%. This application is suitable for different scenarios and conditions to quickly determine the relationship between drugs and cells or multicellular aggregates, and the cell samples can be reused. In addition to measuring the active mechanical forces of cells, it can also measure the stiffness (rigidity) at a certain location inside the cell, and combine the two for analysis, improving the accuracy in its intended application scenarios. It can be applied to scenarios with more microscopic changes, further expanding the information on changes in cells and multicellular aggregates, making characterization easier.

Second, the cell mechanical force detection device included in the cell characterization system provided by this application can detect cell mechanical force simply through reflected light. Compared to existing cell mechanical force detection devices, it has the characteristics of high throughput and low cost. Compared to existing TFM and conventional micropillar arrays, the technical solution of this application eliminates the dependence on expensive confocal microscopes, greatly simplifying the operational process. Because it does not require high-resolution imaging through a microscope, high-throughput monitoring of cells can be achieved at a low cost simply by monitoring the intensity of the reflected light. The cell mechanical force detection device is based on the principle that cell mechanical forces cause micropillar deformation, converting the cell mechanical force into an optical signal for detection, which has the characteristics of high precision and high sensitivity. The optical intensity is linearly correlated with the magnitude of the cell mechanical force, allowing for both qualitative and quantitative analysis. It has high single-cell resolution, allowing for real-time monitoring of each cell, and can be combined with other single-cell analysis techniques to measure the heterogeneity of cell responses to drugs. Real-time monitoring: it is fluorescence-free, avoiding the phototoxic effects of lasers on cells, making it suitable for long-term monitoring and for studying the long-term response of cells to drugs. High sensitivity: the deformation signal of the micropillars is amplified through the reflected signal, increasing the sensitivity of deformation monitoring.

Third, this application utilizes the principle of specular reflection to detect the attenuation of reflected light, which effectively amplifies the signal of micropillar deformation. It has been experimentally verified that the same signal can be observed with a 5x objective lens. Paired with a special reading system, it can effectively detect micro/nano-pillar deformation without relying on high-magnification optical objective lenses, thereby greatly reducing system costs and effectively increasing throughput.

Furthermore, the cell mechanical force detection device of this application can simulate the cellular microenvironment; it can mimic the composition and morphology of the extracellular matrix and can meet the needs of a wider range of technical scenarios. Again, the cell mechanical force detection device of this application can detect the mechanical forces of multilayered cells, including tumor aggregates, making it applicable to scenarios in drug screening, regenerative medicine, gene editing, precision medicine, organ development, and disease modeling that require the characterization of multicellular aggregates.

Further, in the cell mechanical force detection device of this application, the magnetic metal reflective layer and magnetic materials enable the micropillars to achieve more flexible movement patterns and more precise motion control. In addition to measuring active cell mechanical forces, it can also measure the stiffness (rigidity) at a certain location inside the cell and combine the two for analysis, improving the accuracy in its intended application scenarios. It can be applied to scenarios with more microscopic changes, further expanding the information on changes in cells and multicellular aggregates, making characterization easier. The cell mechanical force detection device employs side waveguide illumination and evanescent wave illumination modes, which improves the signal-to-noise ratio and optical effects.

At the same time, this application can use suitable microenvironments and stimuli to amplify the differences between different cell subtypes. For example, by increasing stiffness, the difference between drug-resistant and non-drug-resistant cells can be amplified, making it easier to classify cells using mechanical force. In addition, this application can use conductive materials and fluorescent materials for operations such as electrical stimulation or photolabeling, and can be combined with microfluidic technology for sample sorting. The range of measurable objects for this application includes various types of multicellular aggregates (such as tumor spheroids) and living tissues, and it can obtain their spatial omics information, achieving a comprehensive and objective reflection of the characteristics of cells or multicellular aggregates (such as tumor spheroids, living tissues).

In summary, this application more directly reveals the behavioral changes of cells or multicellular aggregates under the action of drugs, thereby providing a deeper understanding of the mechanisms of drug resistance. It helps to accurately determine the degree of cell drug resistance with higher sensitivity and specificity, and can more accurately reveal the minute changes in cells under the action of drugs. Moreover, distinguishing drug-resistant cells through cell mechanical force also has the advantages of simple operation and low cost.

## BRIEF DESCRIPTION OF THE DRAWINGS

Figure 1 is a schematic structural diagram of a cell mechanical force detection device according to the first embodiment of the present application;

Figure 2 shows Scanning Electron Microscope (SEM) images of the micropillars (actual objects) of a cell mechanical force detection device according to the first embodiment of the present application;

wherein, a is a top view of the cell mechanical force detection device, and b is a side view of the cell mechanical force detection device;

Figure 3 is a schematic structural diagram of a cell mechanical force detection system related to the ninth embodiment of the present application;

Figure 4 is a schematic structural diagram of a cell mechanical force detection system related to the tenth embodiment of the present application;

Figure 5 shows Scanning Electron Microscope images of micropillars (polydimethylsiloxane) with a light-reflective layer (gold) on the top position in a specific embodiment of the present application; wherein, a is an SEM image of the micropillars; b is an elemental characterization map of the top region of the micropillars; c is an elemental characterization map of the side region (excluding the top region) of the micropillars;

Figure 6 shows images from the seventh embodiment of the present application, where cells adhere to the top of micropillars coated with a cell-adhesive substance (fibronectin), wherein, a is a fluorescence imaging image of cells adhering to a preset pattern formed by a group of micropillars with fibronectin on top, and b is a cell force distribution map calculated from the optical reflection signals measured from the cells adhering to the group of micropillars with fibronectin on top;

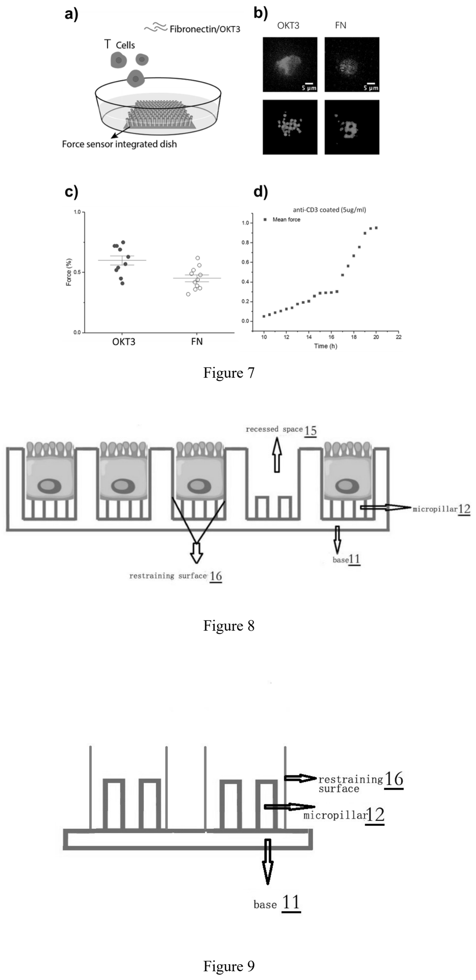

Figure 7 shows images related to an experiment in a specific embodiment of the present application where the cell-adhesive substance used is OKT3 antibody (i.e., a substance that interacts with cell surface receptors) or fibronectin (FN), wherein, a is a schematic diagram of the experiment using OKT3 antibody as the cell-adhesive substance; the two upper images in b are fluorescence imaging images of cells adhering to the tops of micropillars coated with OKT3 antibody and fibronectin, respectively; the two lower images in b are cell mechanical force magnitude distribution maps calculated from the optical reflection signals measured on the micropillars; c is a comparison chart of the force magnitudes measured on surfaces coated with OKT3 antibody and fibronectin, respectively; d is a graph showing the dynamic changes in cell mechanics after T cells were seeded on the OKT3 antibody surface (top of the micropillars);

Figure 8 is a schematic structural diagram of a cell mechanical force detection device with a cell confinement mechanism according to the present application;

Figure 9 is a schematic structural diagram of another cell mechanical force detection device with a cell confinement mechanism according to the present application;

Figure 10 shows images related to a cell mechanical force detection device using a silicon thin film as the cell confinement mechanism in a specific embodiment of the present application, where a is a photograph of the actual device; b is a fluorescence microscope image of the cell mechanical force detection device using a silicon thin film as the cell confinement mechanism under light reflection; c is a magnified view of b;

Figure 11 is a fluorescence microscope image of a cell mechanical force detection system monitoring cell mechanical force in the eleventh embodiment;

Figure 12 is a schematic diagram of the cell mechanical force detection system and its detection results provided in the twelfth embodiment of the present application, where a is a schematic structural diagram of the cell mechanical force detection system of the twelfth embodiment; b is an image of the optical reflection signal from the cell mechanical force detection device acquired by the optical signal detection device of the twelfth embodiment; c is a visualization of the force magnitude and distribution after processing by the optical signal analysis device of the twelfth embodiment;

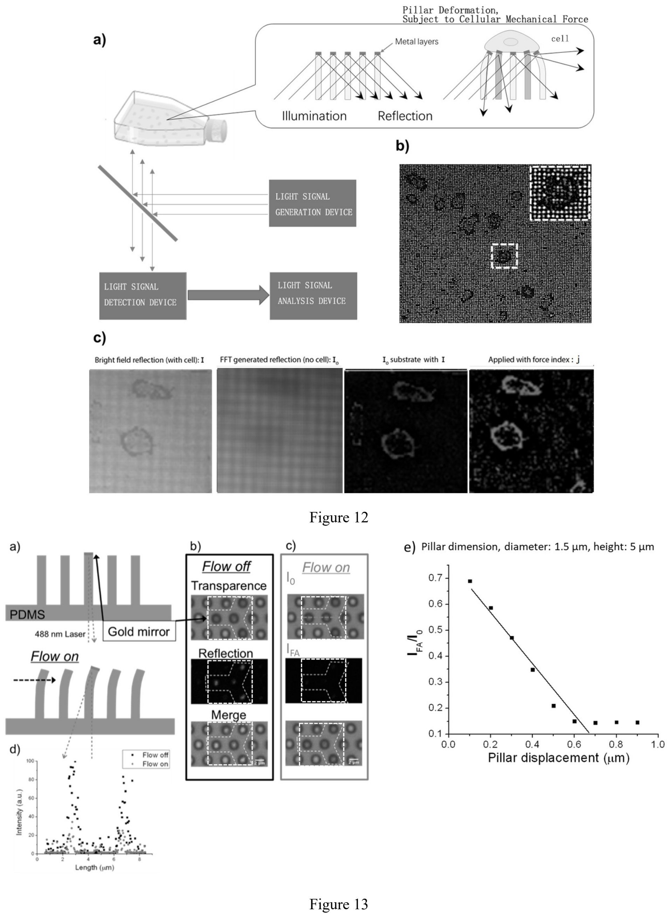

Figure 13 is a schematic diagram illustrating the relationship between cell mechanical force and optical reflection signal using fluid as an external force in a specific embodiment of the present application, where a is a schematic structural diagram of the cell mechanical force detection device in a microfluidic environment before and after the fluid is turned on; b is a comparison of the bright-field microscope image, the reflected light signal distribution map, and their merged effect of the micropillars before the fluid is turned on; c is a comparison of the bright-field microscope image, the reflected light signal distribution map, and their merged effect of the micropillars after the fluid is turned on; in b and c, the merged effect image is the result of overlaying the bright-field microscope image and the reflected light signal distribution map; d is the intensity value of the optical reflection signal before and after the fluid is turned on; e is the linear range of the optical reflection signal attenuation and the displacement of the micropillar tops;

Figure 14 is a schematic diagram of a cell mechanical force detection method according to a specific embodiment of the present application, where a is a schematic structural diagram of the micropillars of the cell mechanical force detection device before and after contact with a cell; b is a reflected light signal distribution map acquired by the optical signal detection device; c is a monitoring graph of the cell migration process; d is a reflected light signal distribution map during the cell migration process;

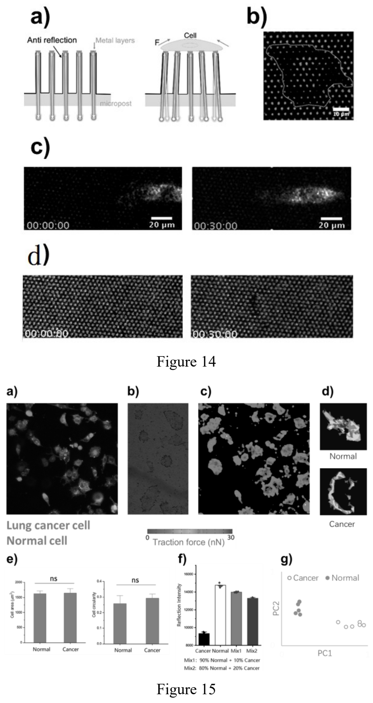

Figure 15 is a schematic diagram illustrating the use of cell mechanical force information obtained by the cell mechanical force detection method of the present application for cell identification, where a is a fluorescence imaging image of a mixed system of healthy cells and non-small cell lung cancer cells; b is a distribution map of the optical reflection signal from the cell mechanical force detection device acquired by the optical signal detection device; c is a visualization of the force magnitude and distribution after processing by the optical signal analysis device; d is a magnified view of the representative single-cell force distribution of healthy cells and non-small cell lung cancer cells in c; e is a comparison of the morphology of healthy cells and non-small cell lung cancer cells; f is a comparison of the reflected signal intensity of healthy cells, non-small cell lung cancer cells, and mixtures of these two cell types in different proportions; g is a cluster analysis diagram obtained by processing c with structuring, based on the structured cell information;

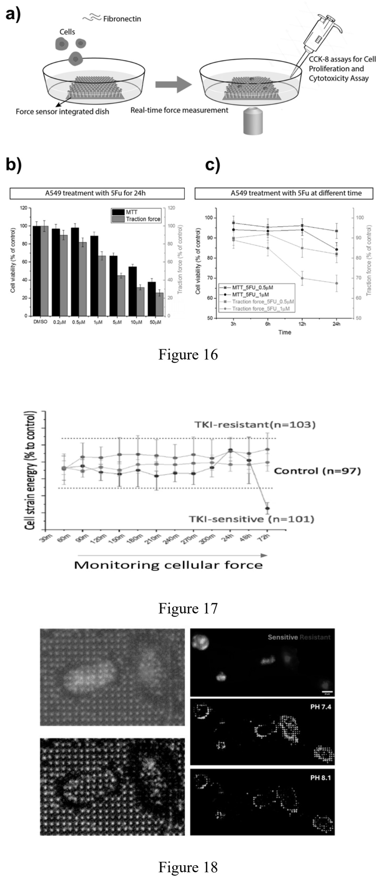

Figure 16 is a schematic diagram illustrating the use of cell mechanical force information obtained by the cell mechanical force detection method of the present application for monitoring cell viability, where a is a schematic diagram of the operational flow of the cell viability detection method; b is a comparison chart of cell viability determined by the MTT assay and cell viability reflected by cell mechanical force after treating A549 cells with different doses of 5FU for 24h; c is a comparison chart of cell viability determined by the MTT assay and cell viability reflected by cell mechanical force after treating A549 cells with different doses of 5FU for different durations;

Figure 17 is a schematic diagram of the dynamic changes in cell mechanical force of cells with different degrees of drug resistance during drug treatment in a specific embodiment of the present application;

Figure 18 shows the distribution and intensity of cell mechanical force at different pH levels in a specific embodiment of the present application;

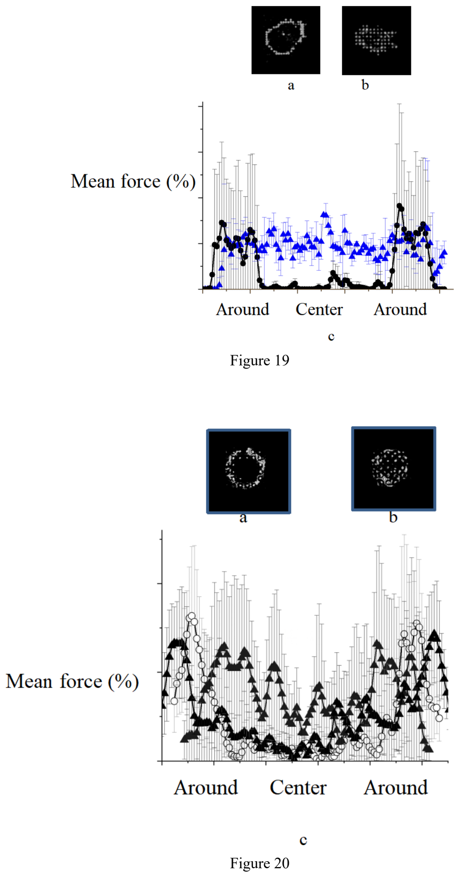

Figure 19 shows the distribution of cell mechanical force intensity for non-small cell lung cancer cells with different degrees of drug resistance and non-drug-resistant cells in a specific embodiment of the present application, where a is a visualized cell mechanical force distribution map of drug-resistant non-small cell lung cancer cells, b is a visualized cell mechanical force distribution map of non-drug-resistant non-small cell lung cancer cells, and c is a distribution graph of the cell mechanical force intensity of drug-resistant and non-drug-resistant non-small cell lung cancer cells, with the horizontal axis representing the radius from the cell center and the vertical axis representing the average cell mechanical force intensity of the corresponding circumference;

Figure 20 shows the distribution of cell mechanical force intensity for breast cancer cells with different degrees of drug resistance and non-drug-resistant cells in a specific embodiment of the present application, where a is a visualized cell mechanical force distribution map of drug-resistant breast cancer cells, b is a visualized cell mechanical force distribution map of non-drug-resistant breast cancer cells, and c is a distribution graph of the cell mechanical force intensity of drug-resistant and non-drug-resistant breast cancer cells, with the horizontal axis representing the radius from the cell center and the vertical axis representing the average cell mechanical force intensity of the corresponding circumference;

Figure 21 is a schematic diagram of a method for sorting EGFR-TKI resistant cells in HCC827 cells based on the distribution of cell mechanical force intensity in the twenty-sixth embodiment of the present application;

Figure 22 is a schematic diagram of the scalarization processing of displacement information at a certain point in the thirty-first embodiment of the present application;

Figure 23 is a result graph of applying the established cell feature model to the identification of unknown cells or unknown cell phenotypes in an extended embodiment of the thirty-second embodiment of the present application;

Figure 24 is a result graph of applying the established cell feature model to the identification of unknown cells or unknown cell phenotypes in an extended embodiment of the thirty-second embodiment of the present application.

## DETAILED DESCRIPTION OF EMBODIMENTS

To better explain and facilitate the understanding of the present application, exemplary embodiments of the present application will be described in more detail below. Although exemplary embodiments of the present application are shown below, it should be understood that the present application may be implemented in various forms and should not be limited by the embodiments set forth herein. Instead, these embodiments are provided so that this disclosure will be clear and thorough, and will fully convey the scope of the application to those skilled in the art.

## First Embodiment

A cell mechanical force detection device.

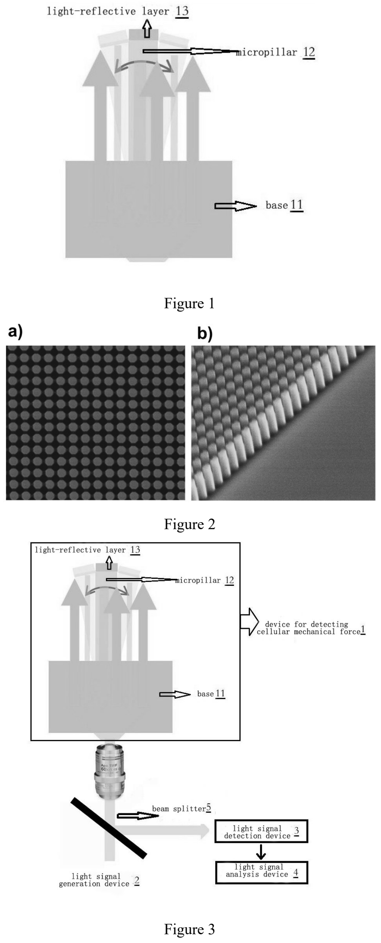

Please refer to Figure 1, which is a schematic structural diagram of a cell mechanical force detection device. The cell mechanical force detection device shown in the figure includes a light-transmissive base 11 and micropillars 12 disposed on the base 11, which can deform under the action of cell mechanical force. The tops of the micropillars 12 are coated with a light-reflective layer 13, and the thickness of the light-reflective layer 13 is 5 nm (in other embodiments, the thickness of the light-reflective layer 13 can be between 5 nm and 20 nm-the thickness of the coating is related to the coating material; assuming the same coating material is used, the selection of the coating thickness should be limited by ensuring the light transmission effect, the stability of the micropillar body, and ensuring that the connection with the micropillar body does not detach). The body of the micropillar 12 can transmit light, and the clusters of arrows in opposite directions in the figure represent incident light and reflected light. (Note: The term "coating" is used in this embodiment only to indicate that the light-reflective layer 13 in this embodiment can be prepared by a coating process, and it does not limit the light-reflective layer 13 to necessarily being prepared by a coating process). Please refer to Figure 2, which shows Scanning Electron Microscope (SEM) images of the micropillars 12 (actual objects) of the cell mechanical force detection device of this embodiment. Figure 2a is a top view of the cell mechanical force detection device, and Figure 2b is a side view. As can be seen from Figure 2, the microstructure of the micropillars of this cell mechanical force detection device is neat and uniform, and the dimensions are controllable. Compared to existing cell mechanical force detection devices, the mechanical values measured by the device of this embodiment are more precise.

When the cell mechanical force detection device 1 of this embodiment is put into use, the number of micropillars 12 will be more than one. Please refer to Figure 3, which is a schematic structural diagram of a cell mechanical force detection system related to the ninth embodiment of the present application; Figure 3 can be used for understanding this embodiment. The system shown in Figure 3 involves, in addition to the cell mechanical force detection device 1 described in this embodiment, an optical signal generation device 2 with a light source and an optical signal detection device 3, both located below the base 11. The light emitted from the light source passes through an incident light path, through the light-transmissive base 11 of the cell mechanical force detection device 1, and illuminates the light-reflective layer on the micropillar 12. The optical signal detection device 3 is used to detect the light reflected from the light-reflective layer 13 on top of the micropillar. The light reflected from the light-reflective layer 13 passes through a reflected light path and enters the optical signal detection device 3 after being acted upon by a beam splitter 5. After obtaining the reflected optical signal, an optical signal analysis device 4 can perform a comparative analysis of the reflected light before and after the cell mechanical force detection device 1 interacts with the cell under test to obtain cell mechanical force information. When the micropillar 12 is not subjected to force, it should remain upright, thus reflecting the probe light to the maximum extent. When the micropillar 12 comes into contact with a cell, it bends under the action of cell mechanical force, leading to a decrease in the level of light reflection. Therefore, the greater the cell mechanical force, the smaller the obtained optical reflection signal should be. In this way, the magnitude of the cell mechanical force at that point can be easily inferred by observing the intensity of the optical reflection signal.

Furthermore, the measurement light source in the technical solution of this embodiment can be an infrared laser of a certain intensity. Traditional micropillar measurement techniques require capturing high-resolution images, and the use of a laser in this process can easily cause cell phototoxicity or sample fluorescence photobleaching. In contrast, if the technical solution herein only needs to measure the reflected signal, the effect of an infrared laser within a certain light intensity on cells is basically negligible, making it suitable for long-term monitoring of cells.

**Second Embodiment**

A cell mechanical force detection device. The difference from the first embodiment is that the micropillar 12 not only has a light-reflective layer 13 on its top end face but also on the upper half of its cylindrical surface (i.e., the curved surface connecting the two end faces of the pillar body). In fact, in other embodiments, except for the solution of placing the light-reflective layer 13 on the lower half of the side cylindrical surface of the micropillar 12, which is not employed due to its poor practical effect, the desired detection effect of the present application can generally be achieved as long as the light-reflective layer 13 is placed on the upper half of the side surface. That is to say, in some other embodiments, the light-reflective layer 13 can even be laid on any local position of the upper half side surface or a local position of the top, and does not necessarily need to cover the entire upper half cylindrical surface or the entire top end face, to achieve the intended purpose, although there may be differences in the acquired data and the effect of subsequent calculations.

Furthermore, the first and second embodiments of the present application have introduced definitions of the "cylindrical surface" and "end face" of the micropillar. That is, a standalone pillar as we usually understand it should have two end faces and a curved surface (cylindrical surface) connecting them. However, due to the presence of the base, the micropillar in this application has only one end face, namely the top end face, while the other end is fixedly connected to or integrally formed with the base. However, in some other embodiments, the top end face may be a smooth, continuous curved surface with the cylindrical surface, without a necessary line of intersection or a clear boundary as shown in the first or second embodiment. In this case, the position of the light-reflective layer 13 will also be understood as being on the upper half of the pillar body and cannot be limited to the "end face" or "cylindrical surface".

**Third Embodiment**

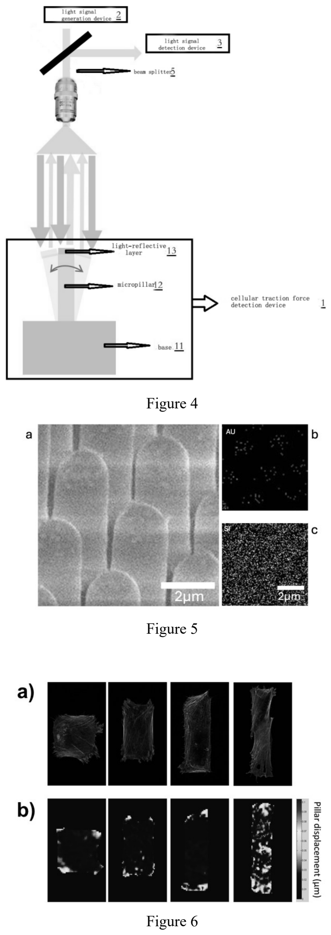

A cell mechanical force detection device. Please refer to Figure 4, which is a schematic structural diagram of a cell mechanical force detection system from the tenth embodiment of the present application, used to illustrate the cell mechanical force detection device 1 of this embodiment. This embodiment differs from the first and second embodiments in that there are no requirements for the light-transmissive properties of the base 11 and the pillar bodies of the micropillar array 12; they can be transparent, non-transparent, or semi-transparent. In this case, it is only necessary to change the positions of the optical signal generation device 2 and the optical signal detection device 3, placing both above the base 11. Thus, each time a micropillar bends, the optical signal received by the optical signal detection device 3 will change relative to when the micropillar 12 is upright and undeformed. By analyzing the changes in the optical signal before and after, the relative magnitude of the cell mechanical force can also be obtained. After calibration with a standard value, the absolute magnitude of the cell mechanical force can be obtained.

**Fourth Embodiment**

A cell mechanical force detection device. This embodiment differs from the first to third embodiments in that an anti-reflective layer for light is provided on the surface of the micropillar 12 in the areas other than where the light-reflective layer 13 is located. This design can reduce the interference of reflected light signals that may come from the surface of the pillar body, enhance the signal-to-noise ratio, and make the detection results more accurate.

In some embodiments, the light-reflective layer 13 can be a layer of gold foil. In other embodiments, the light-reflective layer 13 can also be other metallic layers with light-reflecting functions or other reflective materials. Different materials may result in differences in reflective effect, ease of preparation of the reflective layer, and cost, which can be considered and chosen based on specific conditions in practical operation.

In the first to fourth embodiments, the cross-sectional shape of the micropillars 12 is circular. In other embodiments, the cross-sectional shape of the micropillars 12 can also be elliptical or polygonal. In various specific embodiments of the present application, different cross-sections can achieve different purposes. For example, a circular cross-section has isotropic characteristics, meaning the mechanical properties of the micropillar itself are not sensitive to direction. An elliptical cross-section, however, is anisotropic, meaning the mechanical properties of the micropillar itself are sensitive to direction. This can be used to control the sensitivity to the force field in different directions and can, to a certain extent, regulate the tropism of cells (the geometric morphology of most cells is actually asymmetrical; in this application, the tropism of cells refers to the morphological asymmetry, polarity, or directionality exhibited by the cells. For example, if an ellipse is used to fit the shape of a cell's projection, the major axis of the ellipse can be considered the direction of the cell). Because if the cross-section is elliptical, it has a major axis and a minor axis, it is much easier to push the micropillar along the minor axis than the major axis, thus the deformation under a relative force is also greater. In some extended embodiments, if cells are seeded on such micropillars, there will be an anisotropic mechanical interaction between the cells and the micropillars, which will cause the cells to grow along a certain side. When applied to fluids, this can be used to determine the direction of the fluid.

In the first to fourth embodiments, the dimensions of the micropillar array are: pillar height 10 nm-500 $\mu$m, inter-pillar spacing 10 nm-50 $\mu$m, and pillar top surface diameter 50 nm-50 $\mu$m. Micropillars within this size range can meet the basic operating conditions for use as sensors, i.e., they are at least deformable and do not collapse. On this basis, adjusting the dimensions of different micropillar arrays can also achieve the following functions: for example, by adjusting the Aspect Ratio of the micropillars (which can be understood as the ratio of height to cross-sectional diameter/side length/major axis length at the micropillar level), a certain degree of control over the micropillar's deformation performance can be achieved, thereby better simulating the environment of in vivo organs and tissues (such as bone tissue and nerve tissue of different stiffness).

Furthermore, the overall specifications of the array, or the number of micropillars 12 on a given area of the base 11, will also affect the ligand density, i.e., the number of points on the surface where a cell can find an anchor point for adhesion. If the micropillar 12 array is sparser, the number of adhesion points a cell can find is smaller, which will have a significant impact on cell behavior.

The cross-sectional area of the micropillar's shape will also affect cell adhesion behavior because the formation of Focal Adhesions requires a certain area. If nanometer-scale micropillars are used, the small cross-sectional area of the micropillars will affect the formation of Focal Adhesions.

In summary, by combining the properties of the material itself with specific dimensions of the micropillar array, a more desirable cell support effect, chip stability, and measurement accuracy can be achieved. By adjusting the distribution of the micropillar array, the cell adhesion state can also be regulated and influenced to a certain extent.

In the first to fourth embodiments, the material of the micropillars 12 is polydimethylsiloxane (PDMS). In other main embodiments of this application, the material of the micropillars 12 can also be other polymer materials, such as silicon-based polymers, photoresist polymers, conductive polymers, temperature-sensitive polymers, etc. The main reason for using polymer materials in the main embodiments of this application is that current polymer materials have deformable properties suitable for the application. However, the implementation of this application does not need to limit the micropillar material to polymers; it should and can be extended to all materials with corresponding deformability, all of which can realize the inventive concept of this application. In short, the material of the micropillars must satisfy the condition of having a certain deformability under force, and in some embodiments, it needs to have a certain degree of light transmissivity, although the latter is not a necessary condition for all embodiments. When using materials with limited light-transmissive properties to prepare micropillars, the inventive concept of this application can still be realized by appropriately positioning the optical signal generation device and the optical signal detection device.

Overall, the stiffness (deformability) of the micropillars 12 can be regulated through multiple technical dimensions according to actual needs, including the choice of dimensions (mainly Aspect Ratio) and material type, as well as the control of the degree of cross-linking of the polymer material, and chemical or physical surface treatments.

Please refer to Figure 5. Figure 5 shows Scanning Electron Microscope images of micropillars (polydimethylsiloxane) with a light-reflective layer (gold) on the top position. Wherein, a is an SEM image of the micropillars; b is an elemental characterization map of the top region of the micropillars; c is an elemental characterization map of the side region (excluding the top region) of the micropillars. By characterizing the material composition of the micropillars with the SEM images in Figure 5, it can be confirmed that the element Au is present at the top of the micropillars, and the element Si is present in the remaining parts of the micropillars.

**Fifth Embodiment**

A cell mechanical force detection device. This embodiment differs from the first to fourth embodiments in that the top end faces of some micropillars 12 in the micropillar array are provided with a substance having cell-adhesive properties. This embodiment uses collagen from extracellular matrix molecules. In other embodiments, one or a combination of several extracellular matrix molecules, including collagen, fibronectin, vitronectin, laminin, and tropoelastin, can also be used. In some other embodiments, other types of substances with cell-adhesive properties can be provided on the top end faces of all or part of the micropillars in the micropillar 12 array, such as mimics of the extracellular matrix, like peptides containing the RGD adhesion sequence; or substances with a cell adhesion-promoting mechanism, including poly-L-lysine; or substances that interact with cell surface receptors.

Providing such substances with cell-adhesive properties on the top end faces of the micropillars 12 can effectively promote the attachment of cells to the micropillars 12, thereby achieving regulation of cell attachment, proliferation, migration, state, differentiation, etc. Furthermore, if substances with cell-adhesive properties, such as extracellular matrix proteins like Fibronectin, are provided on the top end faces of some micropillars in a preset area of the micropillar array, these micropillars can form a certain shape. Thus, cells tend to adhere to micropillars in specific positions and shapes, allowing for high-throughput mechanical measurements while controlling cell size, shape, and tropism characteristics.

When the cell mechanical force detection device of this embodiment is applied in an identification method, the cell-adhesive substance can adhere to cells or multicellular aggregates to facilitate the detection of cell mechanical force.

**Sixth Embodiment**

A cell mechanical force detection device. This embodiment differs from the fifth embodiment in that, like the fifth embodiment, the top end faces of some micropillars 12 in the micropillar array are provided with a substance having cell-adhesive properties. In this embodiment, however, the cylindrical surfaces (end face or side surface) of those micropillars 12 whose top end faces are not provided with a cell-adhesive substance are further provided with a substance that inhibits cell adhesion, such as F-127. This makes cells more inclined to adhere to micropillars in specific positions and shapes, thereby enabling high-throughput mechanical measurements while controlling cell size, shape, and tropism characteristics.

**Seventh Embodiment**

A cell mechanical force detection device. This embodiment differs from the first to fourth embodiments in that the micropillars in the micropillar 12 array, whose top end faces are provided with a substance having cell-adhesive properties, form a preset pattern. Specifically, micro-printing technology can be used to print a layer of cell adhesion molecules in a specific pattern to promote cell attachment in these areas. The so-called preset patterns can be shapes like triangles, quadrilaterals, polygons, circles, ellipses, etc. The functions of the preset patterns include: first, controlling cell-to-cell contact through these patterns formed by substances with cell-adhesive properties to facilitate the demand for high-throughput data acquisition. Second, a dimensionality reduction effect in data processing can be achieved by unifying the cell shape, thereby reducing the difficulty of analysis. Third, by restricting the cell attachment area, the size, shape, tropism, and differentiation state of the cells can be controlled. It is even possible to regulate the cell's mechanical state by controlling Actin filaments to meet the requirements of certain special technical scenarios.

In another embodiment largely similar to this one, the areas of the unprinted preset pattern can be coated with a substance that inhibits cell attachment, such as BSA (bovine serum albumin) or F127 (a polymeric non-ionic surfactant), to suppress cell attachment in these areas, thereby achieving directional attachment, control of cell morphology, or simulation of a specific cellular microenvironment.

In some other embodiments, fibronectin (FN) is used as an exemplary substance with cell-adhesive properties, but this is not intended to limit the embodiments of the present application. Using polydimethylsiloxane micro-stamps with raised square and rectangular patterns, fibronectin is adhered to the surface of the micro-stamps. Through micro-contact printing, the fibronectin from the raised parts of the stamp is transferred onto the magnetic metal reflective layer at the top of the micropillars. Next, the micropillars are immersed in an F-127 solution, so that the parts without fibronectin have the effect of inhibiting cell adhesion. Finally, after thoroughly washing the micropillars with saline solution, fibroblasts with stained cell membranes are seeded onto the micropillar surface. Fluorescence imaging of the cells is then performed (as shown in Figure 6a), and high-resolution measurements of the force field within the cells are taken simultaneously (as shown in Figure 6b). Please refer to Figures 6a and 6b. Figure 6a is a fluorescence imaging image of cells adhering to a preset pattern formed by a group of micropillars with fibronectin on top; it can be seen that the cell adhesion is confined to the areas with fibronectin. Based on this, the cell attachment area can be restricted by the preset pattern, and then mechanical monitoring of the cells can be performed while controlling their size, shape, tropism, differentiation state, etc. Figure 6b is a cell mechanical force magnitude distribution map calculated from the optical reflection signals measured on the micropillars.

In some other embodiments, OKT3 antibody (i.e., a substance that interacts with cell surface receptors) or fibronectin (FN) is used as an exemplary substance with cell-adhesive properties, but this is not intended to limit the embodiments of the present application. Please refer to Figures 7a-d. Figure 7a is a schematic diagram of an experiment using OKT3 antibody as the substance with cell-adhesive properties. The upper part of Figure 7b shows fluorescence imaging images of cells adhering to the tops of micropillars coated with OKT3 antibody and fibronectin, respectively. The lower part of Figure 7b shows distribution maps of the optical reflection signal (reflecting the magnitude of cell mechanical force). Figure 7c is a comparison chart of the force magnitudes measured on surfaces coated with OKT3 antibody and fibronectin, respectively. Figure 7d is a graph showing the dynamic changes in cell mechanics after T cells were seeded on the OKT3 antibody surface (top of the micropillars). Specifically, the above experiment can be conducted by coating the tops of some micropillars of the same or different cell force detection devices with OKT3 antibody or fibronectin, and seeding T cells onto the surface of the cell mechanical force detection device with the cell-adhesive substance. As can be seen from Figures 7a-d, a cell mechanical force detection device with its micropillar surfaces coated with a substance that interacts with cell surface receptors (e.g., OKT3 antibody) or fibronectin (FN) can be used for real-time monitoring of the effect of this substance on the cell's mechanical force and their interaction.

**Eighth Embodiment**

A cell mechanical force detection device. This embodiment differs from the first to seventh embodiments in that the cell mechanical force detection device also includes a cell confinement mechanism. The cell confinement mechanism includes one or several confinement surfaces 16. The confinement surface 16 is a plane or curved surface that is perpendicular to the plane where the base 11 is located, connected to the base 11 or integrally formed with the base 11, and the height of the confinement surface 16 is greater than that of the micropillars 12, enclosing a preset number of micropillars 12 within.

The purpose of the cell confinement mechanism set in this embodiment is for single-cell isolation and detection, i.e., to prevent contact or adhesion between cells during detection, and to confine the cell morphology, thereby facilitating high-throughput testing. According to different needs, the number of confinement surfaces 16 or the shape they enclose in the cell position confinement mechanism can be different. For example, the confinement surface 16 included in the cell position confinement mechanism can be a cylindrical surface, or it can be 3 planes connected end-to-end to form a triangular cross-section enclosing a certain number of micropillars, 4 planes perpendicular to each other and connected end-to-end to form a rectangular shape enclosing a certain number of micropillars, N planes connected end-to-end to form an N-sided polygon, or a curved surface with a circular-like cross-section, etc. That is to say, the cross-sectional shape formed by the confinement surfaces 16 is a controllable closed shape, and its area (or understood as the number of micropillars it can accommodate in its space) is also controllable.

In practical embodiments, depending on the manufacturing process, the cell confinement mechanism can also appear in the following forms:

A. Please refer to Figure 8, which is a schematic structural diagram of a cell mechanical force detection device with a cell confinement mechanism. In the figure, the cell confinement mechanism is integrally formed with the base 11, that is: the material forming the cell confinement mechanism has several recessed spaces 15, the wall of the recessed space 15 is the confinement surface 16, the depth of the recessed space 15 is the height of the confinement surface 16, the bottom of the recessed space 15 is the base 11, and each recessed space 15 contains several micropillars 12.

B. Please refer to Figure 9, which is a schematic structural diagram of a cell mechanical force detection device with a cell confinement mechanism. In the figure, the confinement surface 16 is a structure bonded to the base 11.

**Ninth Embodiment**

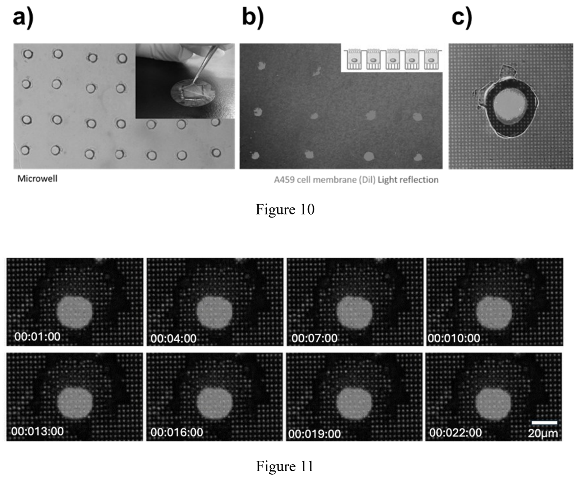

A cell mechanical force detection device. This embodiment differs from the eighth embodiment in that the cell confinement mechanism in this embodiment is a silicon thin film. Specifically, please refer to Figures 10a-c. Figure 10a is a photograph of an actual cell mechanical force detection device using a silicon thin film as the cell confinement mechanism. The silicon thin film, after being perforated by a laser, is adhered to the base. Each hole contains micropillars. The silicon thin film confines the cell's morphology and migration, while also controlling contact or adhesion between cells. Figure 10b is a fluorescence microscope image of the cell mechanical force detection device using a silicon thin film as the cell confinement mechanism under light reflection, and Figure 10c is a magnified view of Figure 10b. In some embodiments, the size of each hole in the silicon thin film can be set to match the size of a single cell, suitable for single-cell attachment, thereby restricting cell contact, cell morphology, and its migration range.

**Tenth Embodiment**

A cell mechanical force detection system, comprising the cell mechanical force detection device 1 of the first or second embodiment, an optical signal generation device 2, and an optical signal detection device 3. Both the optical signal generation device 2 and the optical signal detection device 3 are located below the base 11 in the cell mechanical force detection device 1. The optical signal generation device 2 has a light source. The light emitted from the light source illuminates the light-reflective layer 13 of the micropillar 12 through an incident light path (sequentially passing through the light-transmissive base and the light-transmissive micropillar body), where it is reflected. The reflected light passes through a reflected light path (sequentially passing through the light-transmissive micropillar body and the light-transmissive base) and enters the optical signal detection device 3. The optical signal detection device 3 can acquire the reflected optical signal before and after the micropillar 12 comes into contact with a cell. In some other embodiments, this cell mechanical force detection system also includes an optical signal analysis device 4, which can obtain cell mechanical force information, including the magnitude, direction, and changes over a certain period of time, by comparing, analyzing, and calculating the reflected optical signals before and after the micropillar 12 comes into contact with a cell.

**Eleventh Embodiment**

A cell mechanical force detection system. Please refer to Figure 4. Figure 4 is a cell mechanical force detection system related to the eleventh embodiment of the present application, comprising the cell mechanical force detection device 1 of the third embodiment, and also including an optical signal generation device 2 and an optical signal detection device 3. Both the optical signal generation device 2 and the optical signal detection device 3 are located above the base 11 in the cell mechanical force detection device 1. The optical signal generation device 2 has a light source, the light emitted from the light source illuminates the light-reflective layer 13 through an incident light path and is reflected. The optical signal detection device 3 can acquire the reflected optical signal before and after the micropillar 12 comes into contact with a cell.

In the embodiments of the present application, the optical signal detection device can be a microscope, a charge-coupled device (CCD), a complementary metal-oxide-semiconductor (CMOS), a photomultiplier tube (PMT), a photodetector (PT), photographic film, or other optical signal detection elements with the same function. The present application does not impose specific limitations. It should be noted that in some embodiments of the present application, when a microscope is used as the optical signal detection device, there is no need to set up an independent optical signal generation device. The cell mechanical force detection device of the present application can be placed directly on the microscope stage, using the microscope's light source as the optical signal generation device and the microscope's objective lens (a 5x objective lens is sufficient, no need to rely on high-magnification optical objective lenses) as the optical signal detection device. When other optical signal detection devices are used, such as a charge-coupled device (CCD), an independent optical signal generation device needs to be set up. In the embodiments of the present application, the optical signal generation device can be an LED, a halogen lamp, a laser (for example, an infrared laser), or other light sources, or other devices with these light sources. The present application does not impose specific limitations.

The visualization process of using the cell mechanical force detection system related to this embodiment for cell mechanical force monitoring is specifically introduced below.

Please refer to Figure 11, which shows a fluorescence microscope image of cell mechanical force being monitored by the cell mechanical force detection system of this embodiment. Specifically, cells (for example, this embodiment uses Fibroblasts) are placed on the micropillars of the cell mechanical force detection device. The optical signal detection device (for example, this embodiment uses a microscope) can convert the cell mechanical force information into an optical signal and form an image for visual observation, and can provide real-time feedback on changes in cell mechanical force.

**Twelfth Embodiment**

A cell mechanical force detection system.
Please refer to Figure 4. Figure 4 is a cell mechanical force detection system related to the twelfth embodiment of the present application, comprising the cell mechanical force detection device 1 of the third embodiment, and also including an optical signal generation device 2, an optical signal detection device 3, and an optical signal analysis device 4. Both the optical signal generation device 2 and the optical signal detection device 3 are located above the base 11 in the cell mechanical force detection device 1. The optical signal generation device 2 has a light source, and the light emitted from the light source illuminates the light-reflective layer 13 through an incident light path and is reflected. The beam splitter 5 can be a semi-transmissive, semi-reflective, or other equivalent optical element, mainly to simplify the design of the optical path. The optical signal detection device 3 can acquire the reflected optical signal before and after the micropillar 12 comes into contact with a cell. The optical signal analysis device 4 can obtain cell mechanical force information, including the magnitude, direction, and changes over a certain period of time, by comparing, analyzing, and calculating the reflected optical signals before and after the micropillar 12 comes into contact with a cell.
In the embodiments of the present application, the optical signal analysis device can be optical image analysis software such as ImageJ, Matlab, Fluoview, Python, or other optical image analysis components with the same function, or a combination of these analysis software. The present application does not impose specific limitations.
The detection and analysis process of the cell mechanical force detection system related to this embodiment is specifically introduced below.
Please refer to Figures 12a-c. Figure 12a is a schematic structural diagram of the cell mechanical force detection system; Figure 12b is an image of the optical reflection signal from the cell mechanical force detection device acquired by the optical signal detection device;
Figure 12c is a visualization of the force magnitude and distribution. As shown in Figure 12a, on the cell mechanical force detection device, the top of each micropillar is provided with a magnetic metal reflective layer, and the side surface is provided with an anti-reflective layer. In the absence of cells, light shining from below the micropillars will be completely reflected and fully received by the optical signal detection device (e.g., a CCD camera). However, when cells adhere to the micropillars, the cell force generated by cell movement will cause the micropillars to tilt, thereby reducing the reflected signal. After analysis of the optical reflection signal, the cell force intensity can be calculated.
Further, images of the optical reflection signal of the cell mechanical force detection device and magnified images of local cell adhesion areas are collected by the optical signal detection device (e.g., a CCD camera) (as shown in Figure 12b). Then, the image in Figure 12b is further processed by the optical signal analysis device to convert it into a more intuitive visualization of force magnitude and distribution, Figure 12c.
The specific processing steps are as follows: First, based on Figure 12b, a bright-field reflection signal image (I, focused on the cells) is obtained. Next, by performing a Fourier transform on the image, filtering out high-frequency signals, and then performing an inverse Fourier transform, the reflection signal image of the micropillars in their un-deflected state (I0) is calculated and obtained. Then, the I and I0 images are further processed to convert the reflection signal map into a more intuitive cell force map (I0 signal value minus I signal value), which is then standardized to obtain a more intuitive cell mechanical force intensity map j.

**Thirteenth Embodiment**

Method for calculating mechanical force, and the relationship between force and optical reflection signal.
This embodiment, in conjunction with the cell mechanical force detection system of any one of the tenth to twelfth embodiments or the cell mechanical force detection method of the fourteenth embodiment, explains the method for calculating mechanical force in the embodiments of the present application; and uses a fluid as an external force to verify the relationship between mechanical force and the optical reflection signal.
Please refer to Figure 13, where Figure 13a is a schematic structural diagram of the cell mechanical force detection device in a microfluidic environment before and after the fluid is turned on; Figures 13b and 13c are comparison diagrams of the bright-field microscope images, reflected light signal distribution maps, and their merged effect diagrams of the micropillars before and after the fluid is turned on; d is a graph of the optical reflection signal intensity before and after the fluid is turned on; e is a graph showing the linear relationship between the optical reflection signal and the micropillar displacement. The merged effect diagram refers to the image created by overlaying the bright-field microscope image and the reflected light signal distribution map of the micropillars.

First, as shown in Figure 13, the cell mechanical force detection device is integrated into a microfluidic channel. When the external flow rate increases, the micropillars are displaced, simultaneously changing the angle of the reflective layer on the micropillar surface (Figures 13a-c). As can be seen from Figure 13d, before and after the fluid is turned on, the optical reflection signal changes from strong to weak. Specifically, the displacement of the micropillars is varied by changing the flow rate. A confocal microscope is used to capture the displacement of the top of the micropillar relative to its base. The mechanical force experienced by each micropillar can then be calculated using the following formula:

$$F = k_{\mathrm{bend}}\delta = \frac{3}{64}\pi E \frac{D^4}{L^3}\delta$$

Wherein, F represents the mechanical force causing the micropillar to deflect by an angle δ, E represents the Young's modulus, kbend represents the ideal spring constant of an isolated nanopillar, D represents the diameter of the micropillar, and L represents the height of the micropillar.

At the same time, the optical reflection signal from the top of the micropillar is recorded. By plotting the optical reflection signal against the micropillar displacement as in Figure 13e, a graph showing the linear relationship and linear range between the optical reflection signal (reflecting cell mechanical force) and the micropillar displacement can be obtained.

**Fourteenth Embodiment**

**A Method for Detecting Cell Mechanical Force**

A method for detecting cell mechanical force, comprising the following steps:
Using the optical signal generation device 2 from the cell mechanical force detection system of any one of the tenth to twelfth embodiments to emit light.

Using the optical signal detection device 3 from the cell mechanical force detection system of any one of the tenth to twelfth embodiments to detect the light after it has interacted with the cell mechanical force detection device 1. The optical signal detection device 3 can acquire the reflected optical signal before and after the micropillars 12 of the cell mechanical force detection device 1 come into contact with a cell. In some other embodiments, the optical signal analysis device 4 of the cell mechanical force detection system obtains cell mechanical force information, including its magnitude, direction, and changes over a certain period, by comparing, analyzing, and calculating the reflected optical signals before and after the micropillar 12 comes into contact with the cell.

Please refer to Figures 14a-d. a and b are schematic diagrams of the structure of the micropillars of the cell mechanical force detection device before and after contact with a cell. b shows the reflected optical signal acquired by the optical signal detection device (CCD electronic photosensitive element); a significant attenuation of the reflected signal can be seen in the area of the large force field surrounding the cell. c is a monitoring graph of the cell migration process (after staining the cell membrane, it is excited by a fluorescent light source, and its migration process is recorded by a CCD electronic photosensitive element). d is a distribution map of the reflected optical signal during the cell migration process (the change in the reflected optical signal during its migration is recorded by a CCD electronic photosensitive element). As can be seen from c and d, the reflected optical signal is significantly attenuated in the parts where the cell exerts force. d shows the real-time monitoring of the reflected optical signal during cell migration, and the mechanical force during the migration process is fed back in real-time through the reflected optical signal. It can be seen that monitoring the reflected optical signal during the cell migration process with an optical signal detection device can provide real-time feedback on the changes in cell mechanical force during migration.

**Fifteenth Embodiment**

**A Method for Preparing a Cell Mechanical Force Detection Device**

A method for preparing a cell mechanical force detection device, comprising the following step: laying a light-reflective layer 13 on the top or upper half of the cylindrical surface of the micropillar 12 to obtain micropillars 12 with a reflective layer on the top or upper half of the cylindrical surface.

**Sixteenth Embodiment**

**A Method for Preparing a Cell Mechanical Force Detection Device**

A method for preparing a cell mechanical force detection device, comprising the following steps: uniformly coating the entire micropillar 12 with a layer of anti-reflective coating;

removing the anti-reflective layer from the top or upper half of the cylindrical surface; laying a light-reflective layer 13 on the top or upper half of the cylindrical surface of the micropillar 12.

**Seventeenth Embodiment**

29　　　　**EP 4 692 300 A1**　　　　30

### A Method for Preparing a Cell Mechanical Force Detection Device

This embodiment differs from the fifteenth and sixteenth embodiments in that the step "laying a light-reflective layer 13 on the top or upper half of the cylindrical surface of the micropillar 12" is specified as: uniformly sputtering a layer of reflective metal on the top or upper half of the cylindrical surface of the micropillar to obtain micropillars with a metallic light-reflective layer on the top or upper half of the cylindrical surface. The cell mechanical force detection device of the first to seventeenth embodiments of the present application can be used to detect multicellular aggregates; wherein, the micropillars can be deformed by the cell mechanical force of the multicellular aggregates. The cell mechanical force of multicellular aggregates includes: cell mechanical force, mechanical force generated between cells and the extracellular matrix, and the manifestation of multiple cell mechanical forces.

### Eighteenth Embodiment

### A Cell Identification and Classification System and Its Method for Identifying Cell Types Based on Drug Sensitivity (including qualitative visual identification, and precise qualitative and quantitative identification)

This embodiment specifically provides the use of cell mechanical force information, obtained from the cell mechanical force detection system of any one of the tenth to twelfth embodiments or the detection method of the fourteenth embodiment, applied to a method for identifying cells.

Please refer to Figures 15a to 15g. a is a fluorescence imaging image of a mixed system of healthy cells and non-small cell lung cancer cells; b is a distribution map of the optical reflection signal from the cell mechanical force detection device acquired by the optical signal detection device; c is a visualization of the force magnitude and distribution; d is a magnified view of the representative single-cell force distribution of healthy cells and non-small cell lung cancer cells from c; e is a comparison of the morphology of healthy cells and non-small cell lung cancer cells; f is a comparison of the reflected signal intensity of healthy cells, non-small cell lung cancer cells, and mixtures of these two cell types in different proportions; g is a cluster analysis diagram obtained by processing c with structuring, based on the structured cell information.

Specifically, this embodiment uses healthy cells (Normal) and a non-small cell lung cancer cell line (Cancer) as detection objects. The cell membranes of the healthy and lung cancer cells are pre-stained with two different fluorescent dyes (Dil & DIO), mixed in a certain proportion, and then added to the same cell mechanical force detection device (in other embodiments, they can be added to different, separate cell mechanical force detection devices).

Further, an image of the optical reflection signal of the cell mechanical force detection device was collected by an optical signal detection device (a microscope was used in this embodiment) (as shown in Figure 15b). The high-resolution force field distribution within the two types of cells can be directly rendered by the optical signal detection device and converted into a readable light intensity attenuation signal (reflecting cell force intensity) and displayed in the image (as shown in Figure 15c). Based on the different degrees of light attenuation of the two types of cells shown in c, the two types of cells can be intuitively distinguished by the naked eye (qualitative analysis).

Further, the optical reflection signal in Figure 15c is further processed by an optical signal analysis device. Specifically, this embodiment uses an optical signal analysis device (ImageJ and Python analysis software were used in this embodiment; other image analysis software can be used in other embodiments) to collect information from the obtained cell force field map in Figure 15c. This includes collecting cell mechanical force magnitude information at multiple points for each cell, thereby obtaining multi-point cell mechanical force magnitude data for multiple cells. The acquired cell mechanical force magnitude information is pre-processed to form structured cell information. Based on the analysis of the structured cell information, a comparison result graph of the morphology of healthy cells and non-small cell lung cancer cells is obtained (as shown in Figure 15e).

The structured cell information includes the number of cells, the number of cell features, and the feature information of each cell feature (for example, cell adhesion area and cell circularity in this embodiment). At this point, the structured cell information can be regarded as a two-dimensional feature matrix, where N is the number of cells and P is the number of cell features. Here, P=2, and the cell features are: cell mechanical force magnitude, and the distribution of cell mechanical force within the cell.

Further, using the structured cell information described above as input data, a supervised machine learning model (compared with pre-staining the two cell lines with different cell membrane dyes, Dil & DIO) is used to establish a cell feature model. The cell feature model is trained using the structured cell information from a large number of cells to obtain a cluster analysis graph as shown in Figure 15g. The obtained cell feature model is then applied to the classification and identification of cells of unknown type or unknown state. It can be seen from this that, using structured cell feature data (cell mechanical force magnitude, distribution of cell mechanical force within the cell) as input data, an optical signal analysis device (ImageJ and Python analysis software were used in this embodiment; other cluster analysis software can be used in other embodiments) can be used to perform cluster classification of normal healthy cells

16

and cancer cells, thereby achieving the identification of unknown cell types.

Figure 15e shows that there is no statistically significant difference in the morphology of the different cells (including cell adhesion area and cell circularity). Figure 15f shows a significant difference in the reflected signal intensity (reflecting cell force) between normal cells and tumor cells, and that the reflected signal intensity is linearly related to the mixing ratio when normal and tumor cells are mixed in certain proportions. This shows that, compared to other cell features (such as morphological information like cell adhesion area and cell circularity in e), the cell mechanical features measured by the cell mechanical force detection device of the present application can provide a more intuitive and accurate identification (quantitative and qualitative analysis) of the state and type of cells.

Furthermore, the data from Figures 15d and 15f show that tumor cells exhibit higher mechanical force magnitude than normal cells, and their distribution is more uneven. It is evident that once the cell mechanical force is visualized as an image, the distinct features of the force fields of different cells can be intuitively seen by the naked eye. Furthermore, by structurally processing the force field magnitude at various points of different cells using image analysis software, a comprehensive analysis yields the cell morphology information of Figure 15e, the reflected signal intensity (reflecting cell force) of Figure 15f, and the cluster analysis graph of Figure 15g. Through the comprehensive analysis of the structured information of the force field at various points of the cells, the present application can perform cluster classification and quantitative analysis on different cells (for example, healthy cells and non-small cell lung cancer cells in this embodiment), thereby achieving accurate identification of cell types.

In summary, based on the cell mechanical force detection device of the present application, not only can different cell types be intuitively distinguished by the naked eye for qualitative analysis, but also a more intuitive and accurate identification (quantitative and qualitative analysis) of the state and type of cells can be performed based on the measured cell mechanical features. This confirms that the cell force field serves as a better biomarker for distinguishing cell types.

The cell identification method described above is applicable to the identification of drug-resistant and non-drug-resistant cells.

**Nineteenth Embodiment**

**A Method for Detecting Cell Viability**

This embodiment specifically provides the use of cell mechanical force information, obtained from the cell mechanical force detection system of any one of the tenth to twelfth embodiments or the detection method of the fourteenth embodiment, applied to monitoring cell viability.

Please refer to Figures 16a-c. Figure 16a is a schematic diagram of the operational flow of the cell viability detection method. Figure 16b is a comparison chart of cell viability determined by the MTT assay and cell mechanical force measured by the device, system, or method of the present application after treating A549 cells with different doses of 5FU for 24h. Figure 16c is a comparison chart of cell viability determined by the MTT assay and cell mechanical force measured by the device, system, or method of the present application after treating A549 cells with different doses of 5FU for different durations.

Specifically, in this embodiment, non-small cell lung cancer A549 cells were cultured on multiple cell mechanical force detection devices and treated with different doses of the cell proliferation inhibitor 5-fluorouracil (5-FU). The cell mechanical force at different time points was monitored using the cell mechanical force detection system of any one of the tenth to twelfth embodiments or the detection method of the fourteenth embodiment. Cell proliferation and cytotoxicity at different time points were monitored using a CCK-8 kit. At the same time, cell viability determined by the MTT assay was used as a control group, yielding the data in Figures 16b and 16c.

As shown in Figures 16b and 16c, after measurement by the traditional MTT assay and the device, system, or method of the present application, both the cell viability measured by the MTT assay and the cell viability reflected by cell mechanical force show a dose-dependent decreasing trend, indicating that cell mechanical force is positively correlated with cell viability.

Furthermore, as shown in Figure 16b, after treatment with different doses of 5FU for 24h, compared to the DMSO control group, mechanical force can reflect the decrease in cell viability with a greater magnitude of reduction, thus allowing for a more intuitive assessment of cell viability. As shown in Figure 16c, within 12h of treatment with 5FU, the change in cell viability measured by the MTT assay is not obvious. However, by measuring mechanical force, a decrease in cell mechanical force can be observed at an earlier time point before the MTT assay can detect a decrease in cell metabolic activity. Specifically, a significant decreasing trend appears at 6h with a $0.5\ \mu$M treatment dose, and at 3h with a $1\mu$M treatment dose, thus allowing for a more sensitive characterization of the decrease in cell viability.

In summary, this embodiment demonstrates that directly detecting cell mechanical force with a cell mechanical force detection device is a highly sensitive and effective method for assessing cell viability in response to drugs.

**Twentieth Embodiment**

**A Method for Acquiring Cell Mechanical Force of Cells with Different Degrees of Drug Resistance**

This embodiment provides a method for acquiring cell mechanical force information of cells with different degrees of drug resistance using the cell mechanical force detection device or system from any of the preceding embodiments or the detection method from any of the preceding solutions. Specifically, it includes the following steps:

S1: Obtain several original cells, acquire the cell mechanical force information for each original cell, and record the spatial position of each cell in the cell mechanical force detection device as a marker.

S2: Perform drug screening on the original cells to obtain cells with different degrees of drug resistance. This can be done by directly adding the drug to the cell mechanical force device, or by placing the device in a culture medium containing the drug. The drug can be added periodically and quantitatively. The change in cell mechanical force can, but does not have to, be monitored in real-time by an optical signal detection device and an optical signal analysis device (a microscope can be used as the optical signal detection device for visual monitoring). As shown in Figure 17: Dynamic changes in cell mechanical force during drug treatment show that drug-resistant cells fluctuate within a stable range, while sensitive cells first decrease, then increase, and then suddenly decrease.

S3: Using the spatial position information, the drug resistance information of the cells with different degrees of resistance is matched one-to-one with the original cells before drug screening in step S1. The cell mechanical force information of the cells with different degrees of drug resistance is compared with that of the corresponding original cells. Cells whose cell mechanical force decreases by less than a first preset threshold during and/or after drug treatment are identified as drug-resistant cells, while those whose force decreases by more than or equal to a second preset threshold are identified as non-drug-resistant cells. It should be noted that the aforementioned first preset threshold (e.g., 3%-50%) and second preset threshold are determined based on the actual cells and drugs being tested.

The inventors found in their research that the stronger the drug resistance, the less damage the cell sustains from the drug, and the better its viability, which is manifested as stronger cell mechanical force. The weaker the drug resistance, the greater the change in cell mechanical force. In step S2, the acquisition of cell mechanical force of cells with different degrees of drug resistance by monitoring the change in cell mechanical force can also be verified by methods such as fluorescent labeling. The method for screening cells with different degrees of drug resistance in step S2 can also involve pre-obtaining cells with known corresponding drug resistance levels from the tissues of patients with different degrees of resistance (e.g., highly resistant patients and non-resistant patients). The cell mechanical force information of these cells with different resistance levels and non-resistant cells is initially acquired using the cell mechanical force detection device. Based on this, by acquiring the cell information of the cells to be tested in step S2 and performing comparative analysis, the drug resistance level of each cell can be distinguished. The accuracy of this method for distinguishing cells with different degrees of drug resistance is over 98%.

In some other embodiments, a method for acquiring cell physical information based on drug sensitivity, where the cell physical information includes cell stiffness, comprises the following steps:

S1: Obtain several original cells, acquire the cell physical information for each original cell, and mark the spatial position of each original cell in the cell mechanical force detection device.

S2: Use the same procedure as above to perform specific drug screening on the original cells to obtain cells with different degrees of drug resistance.

S3: Acquire the cell physical information of the cells with different degrees of drug resistance. Using the marked spatial position information, match the drug resistance information of the cells with different resistance levels with the original cells from step S1 before drug screening.

Compare the cell stiffness of the cells with different degrees of drug resistance with that of the corresponding original cells. Cells whose cell stiffness decreases by less than a certain specific preset threshold during and/or after drug treatment are identified as drug-resistant cells, while those whose stiffness decreases by more than or equal to another specific preset threshold are identified as non-drug-resistant cells. The aforementioned specific thresholds are determined based on the actual cells and drugs being tested.

Based on the results for drug-resistant and non-drug-resistant cells, further acquire the cell physical information of the drug-resistant and non-drug-resistant cells for the specific drug.

The same method can be used to acquire the cell mechanical force of multicellular aggregates.

In some specific embodiments, the system also includes a magnetic material device. The micropillars are provided with a magnetic induction material, and the magnetic device acts on the magnetic induction material. The action of the magnetic device on the magnetic induction material causes the micropillars to move or oscillate in a specific direction, as well as deform, causing the micropillars to pierce into a cell or multicellular aggregate to measure cell stiffness. Optionally, the cell mechanical force device is also provided with a diamagnetic material. Optionally, the magnetic device statically or dynamically controls the movement and stiffness of the micropillars via the magnetic induction material. Optionally, the magnetic induction material is a magnetic metal reflective layer or a reflective layer with magnetic properties provided on the surface of the micropillars. Optionally, the magnetic induction material includes one or a combination of two or more of iron, cobalt, nickel, and ferrite. On this basis, cell stiffness information can also be acquired. The cell information, including cell mechanical force information and cell stiffness information, is used for the identification of cells of unknown classification based on drug sensitivity.

**Twenty-first Embodiment**

**A Method for Identifying Cells with Different Degrees of Drug Resistance**

This embodiment provides a method for acquiring cell mechanical force information of cells with different degrees of drug resistance using the cell mechanical force detection device or system from any of the preceding embodiments or the detection method from any of the preceding solutions.

Specifically, it provides a method for identifying EGFR-TKI lung tumor cells with different degrees of drug resistance and non-drug-resistant cells, with the step: determining whether a cell has a different degree of drug resistance based on whether its cell mechanical force is not concentrated around the cell membrane.

In some specific embodiments, the steps may be:

S1: After obtaining tumor tissue from a lung cancer patient, the tissue is minced, and then collagenase and dispase are used to digest the tissue and obtain primary tumor cells.

S2: The cell mechanical force information of the obtained primary tumor cells is acquired using the cell mechanical force detection device from any of the preceding embodiments. The cell mechanical force information can be an image of the optical reflection signal from the cell mechanical force detection device collected by an optical signal detection device (a microscope is used in this embodiment). The high-resolution force field distribution within the two types of cells can be directly rendered by the optical signal detection device and converted into a readable light intensity attenuation signal (reflecting cell force intensity) and displayed in the image. Based on the different degrees of light attenuation of the two types of cells shown in the image, they can be intuitively distinguished by the naked eye (qualitative analysis). The cell mechanical force distribution of primary tumor cells from patients sensitive to EGFR-TKI drugs is compared with that from patients with different degrees of resistance to EGFR-TKI. The cell mechanical force of tumor cells with different degrees of drug resistance is scattered and distributed throughout the entire cell, while the mechanical force of drug-sensitive tumor cells is concentrated around the cell membrane.

The cell mechanical force information can be further processed by an optical signal analysis device on the optical reflection signal from the optical signal detection device. Specifically, this embodiment uses an optical signal analysis device (ImageJ and Python analysis software were used in this embodiment; other image analysis software can be used in other embodiments) to collect information from the obtained cell force field map. This includes collecting cell mechanical force magnitude information at multiple points for each cell, thereby obtaining multi-point cell mechanical force magnitude data for multiple cells. The acquired cell mechanical force magnitude information is pre-processed to form structured cell information. Based on the analysis of the structured cell information, a comparison result graph of the morphology of cells with different resistance levels and non-resistant cells is obtained.

The structured cell information includes the number of cells, the number of cell features, and the feature information of each cell feature (for example, cell adhesion area and cell circularity in this embodiment). At this point, the structured cell information can be regarded as a two-dimensional feature matrix, where N is the number of cells and P is the number of cell features. Here, P=2, and the cell features are: cell mechanical force magnitude, and the distribution of cell mechanical force within the cell. Using the structured cell information described above as input data, a supervised machine learning model (compared with pre-staining the two cell lines with different cell membrane dyes, Dil & DIO) is used to establish a cell feature model. The cell feature model is trained using the structured cell information from a large number of cells to obtain a cluster analysis graph. The obtained cell feature model is then applied to the identification of the different degrees of drug resistance and non-resistance of the tumor cells to be tested. This method can achieve automated identification of cells with different degrees of drug resistance and non-drug-resistant cells in a short period.

**Twenty-second Embodiment**

**A Method for Identifying Cells with Different Degrees of Drug Resistance**

This embodiment provides a method for acquiring cell mechanical force information of cells with different degrees of drug resistance using the cell mechanical force detection device or system from any of the preceding embodiments or the detection method from any of the preceding solutions; and distinguishing cells with different degrees of drug resistance based on the cell mechanical force information.

Specifically, it includes:

S1: Acquiring visual cell information of cells and multicellular aggregates with different degrees of drug resistance based on a specific drug, using a cell mechanical force detection device. This includes visual information such as the magnitude and distribution of changes in cell mechanical force. The drug resistance information for different levels can be obtained from patients with known resistance or verified by other methods.

S2: Using the visual information for the visual identification of the cells and multicellular aggregates to be tested.

**Twenty-third Embodiment**

**A Method for Identifying Cells with Different Degrees of Drug Resistance**

This embodiment provides a method for acquiring cell mechanical force information of cells with different degrees of drug resistance using the cell mechanical force detection device or system from any of the preceding embodiments or the detection method from any of the preceding solutions; and distinguishing cells with different degrees of drug resistance based on the cell mechanical force information.

Specifically, it includes:

S1: Acquiring cell information of cells and multicellular aggregates with different degrees of drug resistance based on a specific drug, using a cell mechanical force detection device. The cell information includes cell mechanical force information at a certain point in the cell acquired by the cell mechanical force detection device. The cell mechanical force information includes the magnitude of the cell mechanical force at that point. Specifically: using an optical signal detection device (or in conjunction with an optical signal analysis device) to collect cell information for multiple cells on the cell mechanical force detection device. This includes collecting cell mechanical force magnitude information at multiple points for each cell, thereby obtaining multi-point cell mechanical force magnitude data for multiple cells.

S2: Pre-processing the acquired cell information of the cells with different degrees of drug resistance to form structured cell information. The structured cell information includes the number of cells, the number of cell features, and the feature information of each cell feature. At this point, the structured cell information can be regarded as a two-dimensional feature matrix, where N is the number of cells and P is the number of cell features. Here, P=1, and the cell feature is cell mechanical force magnitude.

S3: Using the structured cell information as input data, establishing a cell feature model using supervised, unsupervised, or semi-supervised machine learning, and applying the cell feature model to the classification or clustering of the cells to be tested for different degrees of drug resistance, to achieve the identification of cells with different degrees of drug resistance and non-drug-resistant cells.

In some other embodiments of the method for identifying cells with different degrees of drug resistance, the cell mechanical force information also includes the direction of the cell mechanical force at that point. In some other embodiments, the cell mechanical force information also includes the change in the magnitude or direction of the cell mechanical force at that point over a certain time interval. In some other embodiments, the cell information also includes cell morphology information. In some other embodiments, the cell mechanical force information of known cells with different degrees of drug resistance and non-drug-resistant cells is acquired by the method of the eighteenth embodiment. In some other embodiments, the acquisition of cell mechanical force information of cells with different degrees of drug resistance and non-drug-resistant cells involves: pre-obtaining known cells with different degrees of drug resistance and non-drug-resistant cells from the corresponding tissues of patients with different degrees of drug resistance and non-drug-resistant patients, and initially acquiring the cell mechanical force information of these cells using the cell mechanical force detection device.

Based on the cell mechanical force detection device of the present application, not only can different cell types be intuitively distinguished by the naked eye for qualitative analysis, but also a more intuitive and accurate identification (quantitative and qualitative analysis) of the state and type of cells can be performed based on the measured cell mechanical features. This confirms that the cell force field serves as a better biomarker for distinguishing cell types.

**Twenty-fourth Embodiment**

**A Method for Identifying Cells with Different Degrees of Drug Resistance Based on the Effect of pH**

This embodiment provides a method for acquiring cell mechanical force information using the cell mechanical force detection device or system from any of the preceding embodiments or the detection method from any of the preceding solutions, and distinguishing cells with different degrees of drug resistance and non-drug-resistant cells based on the cell mechanical force information. Specifically, it includes:

S1: After obtaining tumor tissue from a lung cancer patient, the tissue is minced, and then collagenase and dispase are used to digest the tissue into single original cells.

S2: A number of original cells are placed on the aforementioned cell mechanical force detection device and cultured along with the device in a normal culture environment (pH 7.4, 37°C, 5% $CO_2$). After the cells adhere to the device, the original cell mechanical force information for each cell is acquired.

S3: The culture medium is replaced with an alkaline culture medium (pH 8.1, 5% $CO_2$), and the original changes in the mechanical force of each cell are recorded in real-time.

S4: Next, the culture medium is replaced with a normal culture medium, and a 10uM drug, EGFR-TKI, is added to kill the cells. The cell mechanical force information of the dying cells (non-drug-resistant cells) and the surviving cells (cells with different degrees of drug resistance) is recorded in real-time. If the cell mechanical force of a cell disappears, it indicates that the cell is entering apoptosis or is already dead. The time point of apoptosis is recorded to infer the degree of drug resistance of each cell.

S5: From the drug treatment experiment in S4, the degree of drug resistance of each cell can be determined. Comparing this with the cell mechanical force data obtained in S3, it is found that the higher the degree of drug resistance of a cell, the more dramatically its cell mechanical force changes when the pH rises, while the change in cell mechanical force of non-drug-resistant cells is smaller.

S6: The cell mechanical force information obtained above can be used to determine the different degrees of drug resistance based on the dynamic changes in cell mechanical force of unknown cell types during the culture process from acidic to alkaline medium, following the same steps S1-S5.

This embodiment shows that changes in pH can amplify the changes in cell mechanical force, making the identification of different types of cells and multicellular aggregates easier. As shown in Figure 18: The distribution and intensity of cell mechanical force at different pH levels.

**Twenty-fifth Embodiment**

**A Method for Identifying Cells with Different Degrees of Drug Resistance Based on Temperature Change**

This embodiment provides a method for acquiring cell mechanical force information using the cell mechanical force detection device or system from any of the preceding embodiments or the detection method from any of the preceding solutions, and distinguishing cells with different degrees of drug resistance based on the cell mechanical force information. Specifically, it includes:

S1: After obtaining tumor tissue from a lung cancer patient, the tissue is minced, and then collagenase and dispase are used to digest the tissue into single original cells.

S2: A number of original cells are placed on the aforementioned cell mechanical force detection device and cultured along with the device in a normal culture environment (37°C). After the cells adhere to the device, the original cell mechanical force information for each cell is acquired.

S3: The temperature of the culture environment is raised to 45°C, and the changes in the mechanical force of each cell are monitored and recorded in real-time.

S4: Next, the temperature is lowered back to the normal culture temperature (37°C), and a 10uM drug, EGFR-TKI, is added to kill the cells. The cell mechanical force information of the dying cells (non-drug-resistant cells) and the surviving cells (cells with different degrees of drug resistance) is recorded in real-time. If the cell mechanical force of a cell disappears, it indicates that the cell is entering apoptosis or is already dead. The time point of apoptosis is recorded to infer the degree of drug resistance of each cell.

S5: From the drug treatment experiment in S4, the degree of drug resistance of each cell can be determined. Comparing this with the cell mechanical force data obtained in S3, it is found that the higher the degree of drug resistance of a cell, the more dramatically its cell mechanical force changes when the temperature rises, while the change in cell mechanical force of non-drug-resistant cells is smaller.

This embodiment shows that changes in temperature can amplify the changes in cell mechanical force, making the identification of different types of cells and multicellular aggregates easier.

**Twenty-sixth Embodiment**

**A Method for Identifying Drug-Resistant and Non-Drug-Resistant Cells of the Lung Cancer Cell Line HCC827**

This embodiment provides a method for acquiring cell mechanical force information using the cell mechanical force detection device or system from any of the preceding embodiments or the detection method from any of the preceding solutions, and identifying the lung cancer cell line HCC827 as cells with a corresponding degree of drug resistance based on the cell mechanical force information.

S1: Primary tumor cells are obtained by taking tumor tissue from lung cancer patients with different degrees of drug resistance and non-drug-resistant patients, mincing the tissue, and then digesting it with collagenase and dispase.

S2: The primary tumor cells from patients with drug resistance and non-drug-resistant patients are cultured separately on the cell mechanical force detection device. Their cell mechanical force distribution is observed. It is found that the cell mechanical force of tumor cells from patients with different degrees of drug resistance is distributed more evenly throughout the entire cell, while the mechanical force distribution of primary tumor cells from non-drug-resistant patients is mainly concentrated around the cell membrane. As shown in Figures 19a and 19b, which are visualized images of the cell mechanical force distribution acquired by an optical detection device. Further analysis of the distribution maps in Figures 19a and 19b yields the graph in Figure 19c, which is a distribution graph of the cell mechanical force intensity of drug-resistant and non-drug-resistant lung cancer cells. The horizontal axis represents the radius from the cell center, and the vertical axis represents the average cell mechanical force intensity of the circumference corresponding to the radius on the horizontal axis.

S3: A number of lung cancer cell line HCC827 cells with unknown degrees of drug resistance are cultured on the micropillars of the cell mechanical force detection device from any of the preceding embodiments. When the mechanical force distribution and other information of a cell are identified to be similar to the cell mechanical force distribution of cells with a certain degree of drug resistance, it can be identified as a cell with that corresponding degree of drug resistance.

In some specific embodiments,

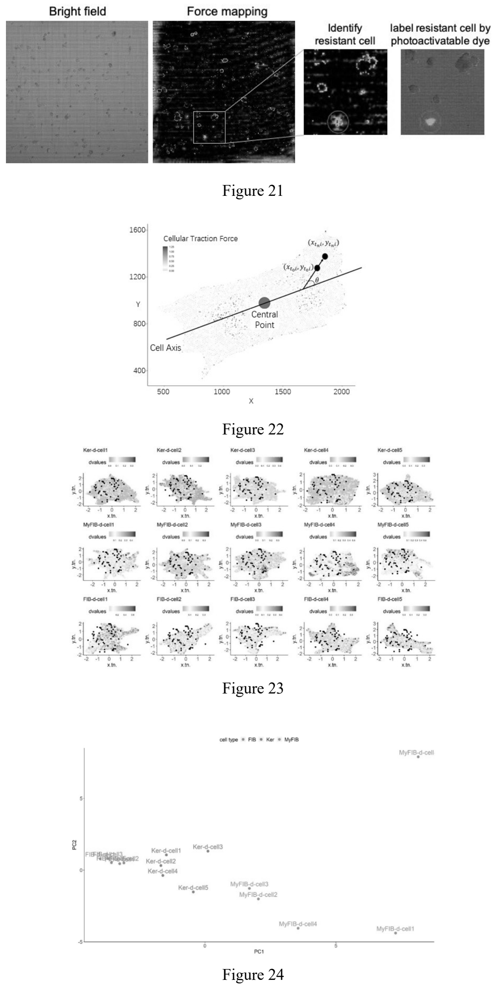

in step S2 of acquiring the cell information of cells with different degrees of drug resistance,

to enable the identification of cells with different degrees of drug resistance in a short period, the lung cancer cell line HCC827 is cultured on the cell mechanical force detection device, and the EGFR-TKI drug is added. The changes in cell mechanical force are monitored in real-time. The top of the micropillars is provided with a culture medium, and the EGFR-TKI drug is added (in some specific embodiments, the drug can be added at regular intervals, for example, once every 3 days). The changes in cell mechanical force can be monitored in real-time by an optical signal detection device and an optical signal analysis device (a microscope can be used as the optical signal detection device for visual monitoring). A photoactivatable fluorescent dye is added, and then the photoactivatable dye is excited by a UV laser to make the cells fluoresce red. Trypsin is used to detach all cells from the cell mechanical force detection device. A flow cytometer is used to sort the fluorescent cells. The sorted cells are cultured in a culture medium containing the EGFR-TKI drug. If they can survive completely, it can be confirmed that the screened cells have the EGFR-TKI resistance feature, as shown in Figure 21.

Figure 19c is a distribution graph of the cell mechanical force intensity of drug-resistant and non-drug-resistant lung cancer cells. The horizontal axis represents the radius from the cell center, and the vertical axis represents the average cell mechanical force intensity of the circumference corresponding to the radius on the horizontal axis.

Please refer to Figure 20. Figure 20a is a visualized cell mechanical force distribution map of drug-resistant breast cancer cells, b is a visualized cell mechanical force distribution map of non-drug-resistant breast cancer cells, and Figure 20c is a distribution graph of the mechanical force intensity of drug-resistant and non-drug-resistant breast cancer cells. The horizontal axis represents the radius from the cell center, and the vertical axis represents the average cell mechanical force intensity of the circumference corresponding to the radius on the horizontal axis.

From the figures, it can be seen that there is a very significant difference in the cell mechanical force of drug-resistant and non-drug-resistant breast and lung cancer cells, which can be quickly qualitatively assessed visually through a microscope. The cell information from Figures 19-20 can be formed into structured information and used for the identification of the drug resistance level of unknown cells.

The method of this embodiment addresses the fact that a single tumor tissue often contains tumor cells with multiple different molecular and phenotypic characteristics, known as intra-tumor heterogeneity, which is significantly related to the varying degrees of resistance to anticancer drugs. Using cell mechanical force distribution to quickly determine whether a patient's primary tumor cells contain tumor cells with different degrees of drug resistance can serve as a predictor of the efficacy of this anticancer drug for the patient. In addition, the screened tumor cells with different degrees of drug resistance can be further tested with other anticancer drugs to achieve personalized medicine.

**Twenty-seventh Embodiment**

**A Method for Identifying Different Degrees of Drug Resistance in A549 Lung Cancer Spheroids**

This embodiment provides a method for acquiring cell mechanical force information using the cell mechanical force detection device or system from any of the preceding embodiments or the detection method from any of the preceding solutions, and identifying lung cancer cell spheroids as having different degrees of drug resistance based on the cell mechanical force information:

S1: Primary tumor cells from patients with different degrees of drug resistance and non-drug-resistant patients are cultured separately on the cell mechanical force detection device. Their cell mechanical force distribution is observed, and a machine learning model is trained using the cell mechanical force database to form a cell feature model. The obtained cell feature model is then used to automatically identify the different degrees of drug resistance of unknown lung cancer cell spheroids.

S2: After obtaining tumor tissue from a lung cancer patient, the tissue is minced, and then collagenase and dispase are used to digest the tissue and obtain primary tumor cells. The tumor cells are cultured in an Ultra-Low Attachment Multiple Well Plate to allow them to grow into multicellular spheroids.

S3: The multicellular spheroids of the lung cancer cell line A549 are cultured on the cell mechanical force detection device, and the EGFR-TKI drug is added. The changes in cell mechanical force are monitored in real-time. When the computer (containing the cell feature model from step S1) identifies that the mechanical force distribution of some cells is approaching that of non-drug-resistant multicellular spheroids (large change in mechanical force), a photoactivatable fluorescent dye is added, and then the photoactivatable dye is excited by a UV laser to make the multicellular spheroids fluoresce red. Next, trypsin is used to detach all multicellular spheroids from the cell mechanical force device and disaggregate them into single cells. A flow cytometer is used to select the fluorescent cells. The sorted cells are re-cultured into multicellular spheroids and then cultured in a culture medium containing the EGFR-TKI drug. If they can survive completely, it can be confirmed that the sorted spheroids are EGFR-TKI resistant cell spheroids. The sorted EGFR-TKI resistant multicellular spheroids are cultured and expanded, then cultured in a multi-well plate containing the cell mechanical force detection device or a general multi-well plate. Next, a compound library is used for drug screening to find drugs that can reverse the mechanical force distribution of the resistant cells or kill the EGFR-TKI resistant multicellular spheroids.

From the above method, it can be seen that the formed cell feature model can be used to identify multicellular spheroids with different degrees of drug resistance in a short period.

**Twenty-eighth Embodiment**

**A Method for Identifying Cells with Different Degrees of Drug Resistance**

This embodiment provides a method for acquiring cell mechanical force information using the cell mechanical force detection device or system from any of the preceding embodiments or the detection method from any of the preceding solutions, and identifying the corresponding degree of drug resistance of unknown cells based on the cell mechanical force information.
This embodiment provides a method for identifying cells with different degrees of drug resistance and non-drug-resistant cells by detecting cell mechanical force with a traction force microscope, with the steps:

S1: A polydimethylsiloxane (PDMS) solution is coated on the bottom of a culture dish. After silanizing the PDMS surface, fluorescent Nanobeads are added on top, and Fibronectin is coated over the nanobeads.

S2: EGFR-TKI cells with different degrees of drug resistance are cultured separately in the culture dishes prepared in the above step. Fluorescent Nanobeads are imaged with a fluorescence microscope, while the cell morphology is captured with a bright-field microscope. An image is taken every fifteen minutes for 24 hours of monitoring. The changes in the positions of the Nanobeads are analyzed to calculate the strength and distribution of the cell mechanical force of the EGFR-TKI cells with different degrees of drug resistance, thereby obtaining their cell mechanical force information.

S3: The same procedure as in step S2 is applied to unknown cells to obtain the cell mechanical force information of cells with different degrees of drug resistance, and a cell feature model is constructed for the automatic identification of the drug resistance level of the unknown cells.

**Twenty-ninth Embodiment**

**A Method for Screening Precision Therapeutic Drugs**

This embodiment provides a method for acquiring cell mechanical force information using the cell mechanical force detection device or system from any of the preceding embodiments or the detection method from any of the preceding solutions, and accurately screening for drugs to which cells are not resistant based on the cell mechanical force information.

The specific steps are:

S1: Obtain the cell mechanical force information of cells with different degrees of drug resistance corresponding to multiple specific drugs, respectively, using the method of the eighteenth embodiment.

S2: Place the cells to be tested on the cell mechanical force detection device (in some other specific embodiments, the micropillars are provided with a culture medium, and the cells are transplanted into the culture medium for culturing). Acquire the cell mechanical force information of the cells to be tested. By comparing with the cell mechanical force information from step S1, the drug resistance situation of the cells to be tested against multiple specific drugs can be quickly determined, achieving precision screening.

Optionally, to achieve AR (Automated Recognition):

S1: Obtain the cell information of cells with different degrees of drug resistance and non-drug-resistant cells corresponding to multiple specific drugs, respectively, using the method of the eighteenth embodiment.

The cell information includes cell mechanical force information at a certain point in the cell acquired by the cell mechanical force detection device. The cell mechanical force information includes the magnitude of the cell mechanical force at that point. Specifically: using an optical signal detection device (or in conjunction with an optical signal analysis device) to collect cell information for multiple cells on the cell mechanical force detection device. This includes collecting cell mechanical force magnitude information at multiple points for each cell, thereby obtaining multi-point cell mechanical force magnitude data for multiple cells.

S2: Pre-process the acquired cell information of the cells with different degrees of drug resistance and non-drug-resistant cells corresponding to different drugs to form structured cell information. The structured cell information includes the number of cells, the number of cell features, and the feature information of each cell feature. At this point, the structured cell information can be regarded as a two-dimensional feature matrix, where N is the number of cells and P is the number of cell features. Here, P=1, and the cell feature is cell mechanical force magnitude.

S3: Using the structured cell information as input data, establishing a cell feature model using supervised, unsupervised, or semi-supervised machine learning, and applying the cell feature model to the classification or clustering of the cells to be tested, to achieve automated identification of the corresponding degree of drug resistance to different drugs, and from there, screen out the drugs to which the cells to be tested have a corresponding degree of drug resistance, achieving precision identification.

In some other embodiments of the method for identifying cells with different degrees of drug resistance and non-drug-resistant cells, the cell mechanical force information also includes the direction of the cell mechanical force at that point. In some other embodiments, the cell mechanical force information also includes the change in the magnitude or direction of the cell mechanical force at that point over a certain time interval. In some other embodiments, the cell information also includes cell morphology information. In some other embodiments, the cell mechanical force information of known cells with different degrees of drug resistance and non-drug-resistant cells is acquired by the method of the eighteenth embodiment. In some other embodiments, the acquisition of cell mechanical force information of cells with different degrees of drug resistance and non-drug-resistant cells involves: pre-obtaining known cells with different degrees of drug resistance and non-drug-resistant cells from the corresponding tissues of patients with different degrees of drug resistance and non-drug-resistant patients, and initially acquiring the cell mechanical force information of these cells using the cell mechanical force detection device.

**Thirtieth Embodiment**

A Method for Identifying Cells Based on Drug Sensitivity (Cell Mechanical Force Magnitude Only, Partially Labeled Data), comprising the following steps:

S1: Acquire the cell information of multiple cells with different degrees of drug resistance and non-drug-resistant cells, where some of the cells are of several known cell types or known cell states, and the rest are of unknown cell types or unknown cell states. The cell information is the magnitude of the cell mechanical force at a certain point in the cell acquired by a cell mechanics sensor. This step specifically includes: using a cell mechanics sensor to collect information for the several types of cells mentioned above, which includes collecting cell mechanical force magnitude information at multiple points for each cell, thereby obtaining multi-point cell mechanical force magnitude data for multiple cells.

S2: Pre-process the acquired cell mechanical force magnitude information to form structured cell information. The structured cell information includes the number of cells, the number of cell features, and the feature information of each cell feature. At this point, the structured cell information can be regarded as a two-dimensional feature matrix, where N is the number of cells and P is the number of cell features. Here, P=1, and the cell feature is: cell mechanical force magnitude.

S3: Using the structured cell information described above as input data, establish a cell feature model using semi-supervised machine learning, and train the cell feature model using the structured cell information from a large number of cells (including both labeled and unlabeled cells). Then, apply the cell feature model to the classification/clustering of cells of unknown type or unknown state.

In other embodiments similar to this one, optimization or improvement can be made in the following way: for a single cell, further information processing is performed on the acquired multi-point cell mechanical force magnitude information, for example, by calculating: the average value of cell mechanical force magnitude per unit area; the distribution of cell mechanical force magnitude within the cell; and other dimensional information. This can serve as new cell features to be added to the two-dimensional feature matrix in step S2, i.e., expanding the content of P. Then, through subsequent machine learning, it can be determined which features can better distinguish between cells with different degrees of drug resistance and non-drug-resistant cell types.

**Thirty-first Embodiment**

A Method for Identifying Cells Based on Drug Sensitivity (Magnitude and Direction of Cell Mechanical Force, Unlabeled), comprising the following steps:

S1: Acquire cell information, where the cell information is the magnitude and direction of the cell mechanical force at a certain point in the cell acquired by a cell mechanics sensor. This specifically includes: using a cell mechanics sensor (a nano-micropillar sensor in this embodiment) to collect information for multiple cells, which includes collecting cell mechanical force magnitude and direction information at multiple points for each cell, thereby obtaining multi-point cell mechanical force magnitude and direction data for multiple cells.

S2: Pre-process the acquired cell mechanical force magnitude and direction information to form structured cell information. The structured cell information includes the number of cells, the number of cell features, and the feature information of each cell feature. At this point, the structured cell information can be regarded as a two-dimensional feature matrix, where N is the number of cells and P is the number of cell features. Here, P=2, and the cell features are: cell mechanical force magnitude, cell mechanical force direction.

S3: Using the structured cell information described above as input data, establish a cell feature model using unsupervised machine learning, and apply the cell feature model to the clustering of cells of unknown type or unknown state.

Specifically, the S2 step in this embodiment processes the cell information roughly as follows: assume that for one cell, a total of n data points of cell mechanical force vectors (magnitude and direction) are collected. These points are:

$i$, $i \in \{1, 2, ...n\}$, respectively corresponding to two two-dimensional coordinates: $(x_{t0i}, y_{t0i})$ and $(x_{tni}, y_{tni})$, wherein $t_0$ and $t_n$ respectively correspond to the nano-micropillar's initial and after-displacement positions. This way, $(x_{tni} - x_{t0i}, y_{tni} - y_{t0i})$ is the direction of the force on each coordinate point. In addition, on each coordinate position there should also be one piece of scalar information, that is the magnitude of the force $d$. Thus it is possible through existing data to estimate the cell axis direction and the center point coordinates, and using this as a baseline, organize each cell into a vector of the same length. For example, based on each point position within each cell, the center point can be calculated as: $\frac{1}{n}\sum_i(x_i, y_i)$.

The calculation method for the cell axis is to find the two points that are furthest apart $(x_1, y_1)$ and $(x_2, y_2)$, and through the following formula, obtain the cell axis:

$$\beta = \frac{y_2 - y_1}{x_2 - x_1}, \ \alpha = y_2 - \beta x_2, \ y = \alpha + \beta x.$$

Please see Figure 22, Figure 22 is a schematic diagram of the scalarization processing of the displacement information of a certain point position in the fourth embodiment of this application, in the figure each dot represents a point position, the color depth of each point position from light to deep represents the force from small to large. After obtaining the cell axis of each cell, each point position can be further quantitatively processed: calculate the angle between the displacement vector of each point and the cell axis as $\theta$. Each point position can then be combined with the magnitude of the force (scalar), for example by treating the magnitude of the force $d$ as a weight, thus processing each point position into a scalar $s_i = \theta \cdot d_i$. This way, the cell information of each cell can all be organized into a vector $z = (s_1, ...s_n)$.

In other embodiments similar to this embodiment, it is possible to optimize or improve in the following ways: for a single cell, perform further information processing on its acquired multi-point cell mechanical force magnitude or cell mechanical force direction information, for example calculating: the average value of cell mechanical force magnitude per unit area; the distribution situation of cell mechanical force magnitude within the cell; the distribution situation of cell mechanical force vectors within the cell; information of other dimensions, this can be used as new cell features, added into the two-dimensional feature matrix in step S2, that is, expanding the content of P, and then through subsequent machine learning, learn which kind of feature can better distinguish cells of different types or states.

## Embodiment 32

A method for identifying cells based on drug sensitivity (magnitude and direction of cell mechanical force, labeled), comprising the following steps:

S1, acquiring cell information of several known cell types or cells in known states, where the cell information is the magnitude and direction of the cell mechanical force at a certain point in the cell, obtained based on a cell mechanics sensor, specifically comprising: using a cell mechanics sensor to collect information on the aforementioned several types of cells, including collecting information on the magnitude of the cell mechanical force at multiple points of each cell, thereby obtaining data on the magnitude of the cell mechanical force at multiple points in multiple cells;

S2, pre-processing the acquired information on the magnitude of cell mechanical force to form structured cell information; the structured cell information includes the number of cells, the number of cell features, and the feature information of each cell feature, at which point the structured cell information can be regarded as a two-dimensional feature matrix, where N is the number of cells, P is the number of cell features, and here P=2, i.e., the cell features are: magnitude of cell mechanical force, direction of cell mechanical force;

S3, using the aforementioned structured cell information as input data, establishing a cell feature model using supervised machine learning, and training the cell feature model using the structured cell information of a large number of cells, then applying the cell feature model to the classification of cells of unknown types or in unknown states. For example, this embodiment adopts the Random Forest (RF) algorithm to extract significant features and estimate model parameters, which are then applied to new cells to estimate the labels corresponding to the new cells, i.e., applying the cell feature model to the classification of cells of unknown types or in unknown states. In other embodiments, algorithms/ideas from machine learning such as Support Vector Machine (SVM) or deep learning may also be adopted to complete the corresponding model establishment and training work.

Please refer to Figure 22 and Figure 23. Figure 22 and Figure 23 are result diagrams A and B, respectively, of applying the established cell feature model to the identification of unknown cells or unknown cell phenotypes in an extended embodiment of the fifth embodiment of the present application. In Figure A, different rows represent different cell types, different columns represent different samples, and the black dots in the figure are the top 50 significant features extracted using the Random Forest algorithm, which can also be called significant point locations. The point locations here refer to a certain position within a cell, and the cell mechanical force information obtained from different positions is different. Figure B, on the other hand, shows the significant distinguishing effect on three different cell types using the top 50 significant features (significant point locations). Significant features learned from labeled data can be used for data dimensionality reduction and visualization. Subsequently, clustering algorithms can also be used to classify and identify cells based on the dimensionally reduced data.

In some other embodiments similar to this embodiment, optimization or improvement can be made in the following manner: for a single cell, further information processing is performed on the acquired multi-point information of the magnitude or direction of the cell mechanical force, for example, calculating information in dimensions such as: the average value of the magnitude of cell mechanical force per unit area; the distribution of the magnitude of cell mechanical force within the cell; the distribution of the cell mechanical force vector within the cell, etc. This can be used as new cell features and added to the two-dimensional feature matrix in step S2, i.e., expanding the content of P, and then learning through subsequent machine learning which features can better distinguish between different types or states of cells.

## Embodiment 33

A method for identifying cells based on drug sensitivity (magnitude and direction of cell mechanical force, some data labeled, some data unlabeled), comprising the following steps:

S1, acquiring cell information of multiple cells, wherein some of the cells are of several known cell types or in known cell states, and the remaining cells are of unknown cell types or in unknown cell states; the cell information is the magnitude of the cell mechanical force at a certain point in the cell, obtained based on a cell mechanics sensor. Specifically comprising: using a cell mechanics sensor to collect information on the aforementioned several types of cells, including collecting information on the magnitude of the cell mechanical force at multiple points of each cell, thereby obtaining data on the magnitude of the cell mechanical force at multiple points in multiple cells;

S2, pre-processing the acquired information on the magnitude of cell mechanical force to form structured cell information; the structured cell information includes the number of cells, the number of cell features, and the feature information of each cell feature, at which point the structured cell information can be regarded as a two-dimensional feature matrix, where N is the number of cells, P is the number of cell features, and here P=2, i.e., the cell features are: magnitude of cell mechanical force, direction of cell mechanical force;

S3, using the aforementioned structured cell information as input data, establishing a cell feature model using semi-supervised machine learning, and training the cell feature model using the structured cell information of a large number of cells, then applying the cell feature model to the classification of cells of unknown types or in unknown states.

In some other embodiments similar to this embodiment, optimization or improvement can be made in the following manner: for a single cell, further information processing is performed on the acquired multi-point information of the magnitude or direction of the cell mechanical force, for example, calculating information in dimensions such as: the average value of the magnitude of cell mechanical force per unit area; the distribution of the magnitude of cell mechanical force within the cell; the distribution of the cell mechanical force vector within the cell; etc. This can be used as new cell features and added to the two-dimensional feature matrix in step S2, i.e., expanding the content of P, and then learning through subsequent machine learning which features can better distinguish between different types or states of cells.

**Embodiment 34**

A method for identifying cells based on drug sensitivity (instantaneous value of the cell mechanical force vector, change of the cell mechanical force vector within a certain time interval, unlabeled), comprising the following steps:

S1, acquiring cell information, where the cell information is the instantaneous value of the vector of the cell mechanical force at a certain point in the cell and the change of the cell mechanical force vector at that point within a certain time interval, obtained based on a cell mechanics sensor; specifically comprising: using a cell mechanics sensor to collect cell information on multiple cells, including collecting information on the magnitude and direction of the cell mechanical force at multiple points of each cell, thereby obtaining data on the magnitude and direction of the cell mechanical force at multiple points in multiple cells;

S2, pre-processing the acquired information on the magnitude and direction of the cell mechanical force to form structured cell information; the structured cell information includes the number of cells, the number of cell features, and the feature information of each cell feature, at which point the structured cell information can be regarded as a two-dimensional feature matrix, where N is the number of cells, and P is the number of cell features.

S3, using the aforementioned structured cell information as input data, establishing a cell feature model using unsupervised machine learning, and applying the cell feature model to the clustering of cells of unknown types or in unknown states.

In this embodiment, since what is acquired is not only the instantaneous value of the cell mechanical force vector at a certain point within the cell, but also its interval situation within a certain time interval, the mechanics data of a single cell obtained can actually be analogized to an image (instantaneous value) or a video (change situation within a time dimension). In this way, if the dot matrix covered by the current cell is analogized to the pixels in an image, and the information recorded at each point (magnitude of force, direction, and other multiple cell features) can be analogized to the colors corresponding to the pixels, then in subsequent machine learning, machine learning algorithms from the field of image and video data processing can be referenced. For example, machine learning widely used in image recognition, more precisely, the Convolutional Neural Network (CNN) in deep learning, can be adopted for data modeling and analysis.

**Embodiment 35**

A method for identifying cells with different degrees of drug resistance (instantaneous value of the cell mechanical force vector, change of the cell mechanical force vector within a certain time interval, labeled), comprising the following steps:

S1, acquiring cell information of several known cell types or cells in known states, where the cell information is the instantaneous value of the vector of the cell mechanical force at a certain point in the cell and the change of the cell mechanical force vector at that point within a certain time interval, obtained based on a cell mechanics sensor; specifically comprising: using a cell mechanics sensor to collect cell information on multiple cells, including collecting information on the magnitude and direction of the cell mechanical force at multiple points of each cell, thereby obtaining data on the magnitude and direction of the cell mechanical force at multiple points in multiple cells;

S2, pre-processing the acquired information on the magnitude and direction of the cell mechanical force to form structured cell information; the structured cell information includes the number of cells, the number of cell features, and the feature information of each cell feature, at which point the structured cell information can be regarded as a two-dimensional feature matrix, where N is the number of cells, and P is the number of cell features.

S3, using the aforementioned structured cell information as input data, establishing a cell feature model using supervised machine learning, and training the cell feature model using the structured cell information of a large number of cells, then applying the cell feature model to the classification of cells of unknown types or in unknown states (i.e., identification; "identification" in this application should be understood in a broad sense, including not only "classification," i.e., determining the type or state of a cell, but also "clustering," i.e., clustering cells that may have the same or similar properties, and may be of the same or similar type or state, even if the specific state or type is unknown). For example, this embodiment adopts the Random Forest (RF) algorithm to extract significant features and estimate model parameters, which are then applied to new cells to estimate the labels corresponding to the new cells, i.e., applying the cell feature model to the classification of cells of unknown types or in unknown states. In other embodiments, algorithms/ideas from machine learning such as Support Vector Machine (SVM) or deep learning may also be adopted to complete the corresponding model establishment and training work.

In this embodiment, since what is acquired is not only the instantaneous value of the cell mechanical force vector at a certain point within the cell, but also its interval situation within a certain time interval, the mechanics data of a single cell obtained can actually be analogized to an image (instantaneous value) or a video (multiple instantaneous values within a certain time dimension). In this way, if the dot matrix covered by the current cell is analogized to the pixels in an image, and the information recorded at each point (magnitude of force, direction, and various other cell features) can be analogized to the colors corresponding to the pixels, then in subsequent machine learning, machine learning algorithms from the field of image and video data processing can be referenced. For example, machine learning widely used in image recognition, more precisely, the Convolutional Neural Network (CNN) in deep learning, can be adopted for data modeling and analysis.

**Embodiment 36**

A method for measuring the physical characteristics, including cell hardness, of a substance and a cell/multicellular aggregate by magnetic force action. This embodiment specifically provides a biophysical characterization apparatus, comprising: a base, and a micropillar array composed of a plurality of micropillars disposed on the base, which can undergo a state change upon the action of magnetic force, the state change comprising: at least one of pulling, movement in various directions, and deformation; the micropillar comprises a bottom end connected to the base, a side surface, a pillar body enclosed by the side surface, and a top end away from the base and opposite the bottom end, a light reflection layer is disposed on the top end and/or the side surface of the micropillar, a magnetic material is disposed at any position on the top end, side surface, and within the pillar body of the micropillar, and preferably, a magnetic light reflection layer is disposed on the top end of the micropillar. The magnetic material in this application refers to a material that reacts to a magnetic field in some way, including but not limited to ferromagnetic materials, paramagnetic materials, diamagnetic materials, ferrimagnetic substances, antiferromagnetic substances, and superparamagnetic materials. The magnetic light reflection layer is a magnetic metal reflection layer.

When the characterization apparatus provided by the present application characterizes a cell or multicellular aggregate, the micropillar can pierce the cell or multicellular aggregate, and the piercing force includes but is not limited to the gravity of the cell or multicellular aggregate, the adhesion force generated on the cell or multicellular aggregate by a substance with a mechanism for promoting cell adhesion, applying pressure on the micropillar or the cell or multicellular aggregate, etc. In particular, after the micropillar pierces the cell or multicellular aggregate, the magnetic metal light reflection layer is acted upon by a magnetic force to cause the micropillar to undergo at least one of pulling, movement in various directions, and deformation.

In some specific embodiments, an anti-reflection layer is disposed on the side surface of the micropillar and/or the surface of the base. This arrangement can significantly improve the signal-to-noise ratio of the biophysical characterization, making the measurement results more accurate. In some specific embodiments, the biophysical characterization apparatus further comprises a plurality of cell confinement structures disposed on the base. In some specific embodiments, at least one of the base, the micropillar, and the confinement surface is provided with a substance that interacts with cells. In some specific embodiments, the substance that interacts with cells is disposed on the top end of the micropillar, and a plurality of micropillars provided with the substance that interacts with cells form a first predetermined pattern; and/or a substance with an anti-cell adhesion effect is disposed on the top end of the micropillar, and a plurality of micropillars provided with the substance with an anti-cell adhesion effect form a second predetermined pattern. The first predetermined pattern may be the same as or different from the second predetermined pattern, and such naming in this application is merely for distinguishing and identifying the patterns formed by the entirety of micropillars provided with the substance that interacts with cells and the substance with an anti-cell adhesion effect, respectively. In some specific embodiments, a fluorescent substance is disposed on an extension part from the top end to the bottom end of the micropillar. The extension part comprises the side surface of the micropillar and the solid part within the pillar body enclosed by the side surface. This arrangement can achieve the purpose of more intuitively calibrating the cell or multicellular aggregate to amplify the signal and improve the signal-to-noise ratio. At the same time, if the cell or multicellular aggregate to be characterized is stained with a fluorescent substance of another color, the hardness of the cell or multicellular aggregate can be judged by the depth of piercing.

This embodiment specifically provides a biophysical characterization system at the same time, comprising: one or more multimodal biophysical characterization apparatuses of the first aspect of the present application; a light signal emitting device, which is used to emit predetermined light; a light signal detecting device, which is used to detect the light reflected from the light reflection layer; and a magnetic field generating device, which is used to generate a magnetic field to produce a magnetic force action with the magnetic material; the light emitted by the light signal emitting device illuminates the light reflection layer through an incident light path, and the light reflected by the light reflection layer enters the light signal detecting device through a reflection light path.

In some specific embodiments, the biophysical characterization system further comprises a light signal analysis device, which is used to analyze the light signal. In some specific embodiments, the biophysical characterization system further comprises a data processing device, which is used to compute and process the light signal to obtain the results of cell mechanical force and/or cell softness/hardness and their spatial distribution. In some specific embodiments, the data processing device can also provide predictive information on cell behavior and differentiation direction based on the results of cell mechanical force and/or cell softness/hardness and their spatial distribution. In some specific embodiments, the characterization system further comprises a pressure application device, which is used to apply pressure to the cells. The pressure application device includes but is not limited to a gravity block, an automated pressure device, a probe, etc. In some specific embodiments, the light signal emitting device has a light source, which includes a first light source disposed at the bottom of the base and/or a second light source disposed at the side of the base, and the light emitted by the first light source and/or the second light source reaches the micropillar. In some specific embodiments, the first light source and the second light source do not imply a limitation on the setting order and type of the light sources, but are merely for distinguishing the light sources at different setting positions. Preferably, the second light source employs a waveguide illumination light source. In some specific embodiments, the biophysical characterization system further comprises a device for causing movement or deformation, which is used to apply a mechanical force to the multimodal biophysical characterization system. The device for causing movement or deformation includes but is not limited to a pulling device or an inflation/deflation device, wherein the pulling device is used to apply an appropriate mechanical pulling force to the base to control the stretching movement or deformation of the base in the horizontal direction, and the inflation/deflation device is used to form a movement or deformation on a curved surface of the base.

In some specific embodiments, the base and/or the micropillar are made of a conductive material. The purpose of using a conductive material for the base and/or the micropillar is, in addition to being able to implement electrical stimulation during the process of characterizing the cell or multicellular aggregate, also to achieve the purpose of receiving the electrical signals of the cells. In some specific embodiments, the biophysical characterization system is constituted by two or more biophysical characterization apparatuses to form a double-sided structure or a multi-sided structure, where the outer side of the double-sided structure or multi-sided structure is the base, and the inner side encloses a three-dimensional accommodation cavity for cells. In some specific embodiments, when two or more multimodal biophysical characterization apparatuses constitute a double-sided or multi-sided structure, the cell confinement structures are adapted to each other. In particular, before, during, or after the physical characterization of the cell or multicellular aggregate, the sample to be tested can also be subjected to stimuli of different types and intensities, such as but not limited to mechanical stimulation, electrical stimulation, light stimulation, etc., and operations such as but not limited to marking, fixing, ablating, cutting, extracting, sorting, etc., can be performed on the sample to be tested at specific positions or regions, and combined with other characterization methods such as but not limited to protein staining, histochemical staining, single-cell sequencing, etc., for comparative analysis.

In some specific embodiments, the micropillar is simultaneously set to be deformable by cell mechanical force, which can be used to obtain the cell mechanical force, and the specific structure, function, and application are as shown in the first to thirty-sixth embodiments; at this time, the magnetic metal reflection layer serves as a light reflection layer, and under the action of a light source, the deformation of the micropillar due to cell mechanical force can also be obtained, converting the cell mechanical force information into an optical signal for the detection of cell mechanical force. That is, when the light reflection layer is a magnetic metal reflection layer, it can not only be used to detect the cell mechanical force, but also, under the action of the magnetic field generating device, to detect the hardness of the cells. The simultaneous detection of mechanical force and hardness can provide a more comprehensive acquisition of the overall information of the cells, improving the accuracy of predicting and judging the typing, growth state, etc., related to the cell/multicellular aggregate. It can be used to determine or predict the interaction between drugs and the cell/multicellular aggregate to perform typing for drug sensitivity; through the changes and dynamic trends of the mechanical force and hardness of the cell or multicellular aggregate, to predict the behavior of the cells, including but not limited to state, differentiation, proliferation, migration, and apoptosis.

In summary, this embodiment can achieve multimodal, high-resolution, high-throughput, high-sensitivity, low-cost, low-damage physical characterization of single cells or multicellular aggregates, and can subject the sample to be tested to stimuli of different types and intensities (such as mechanical stimulation, electrical stimulation, light stimulation, etc.) to better simulate the in vivo microenvironment, as well as perform operations on samples at specific positions or regions (such as marking, fixing, ablating, cutting, extracting, sorting, etc.), and combine with other characterization methods (such as protein staining, histochemical staining, single-cell sequencing, etc.) for comparative analysis. The multimodal biophysical characterization apparatus provided by the present application is suitable for fields such as synthetic biology, diagnostics, drug discovery, early tumor screening, cell therapy, and precision medicine.

Finally, it should be noted that: the above embodiments are only used to illustrate the technical solutions of the present application, not to limit them; although the present application has been described in detail with reference to the foregoing embodiments, a person of ordinary skill in the art should understand that: they can still modify the technical solutions recorded in the foregoing embodiments, or make equivalent substitutions for some or all of the technical features therein; and these modifications or substitutions do not make the essence of the corresponding technical solutions depart from the scope of the technical solutions of the embodiments of the present application.

**Claims**

1. An identification method for cell drug resistance level, **characterized in that** comprising:

   acquiring cell physical information, wherein the cell physical information comprises cell mechanical force, and/cell stiffness;
   identifying cells with different drug resistance levels, as well as non-drug-resistant cells, according to the cell physical information.

2. The identification method according to claim 1, wherein the cell mechanical force comprises one or more of magnitude, direction, distribution, and frequency of the cell mechanical force, and preferably comprises one or more of magnitude, direction, distribution, frequency of the cell mechanical force and a variation over time thereof;

   the cell stiffness comprises magnitude of cell stiffness at specific locations within the cell, and preferably comprises magnitude of stiffness and spatial distribution of different layers at specific locations within the cell, and a variation over time thereof.
   preferably, said cell physical information is obtained before, during, and/or after the action of a specific drug;
   preferably, said cell physical information comprises a cell mechanical force and/or a cell stiffness acquired during at least one of the following: interactions between cells and multicellular aggregates, interactions between cells, interactions between multicellular aggregates, an effect of substances on cells and/or multicellular aggregates, and an effect of other physical, biological, or chemical factors on cells and/or multicellular aggregates.

3. The identification method according to claim 2, wherein the cell physical information comprises a combination of one or more at least one of cell number, cell mechanical force, cell stiffness, and cell morphology.

4. The identification method according to claim 1, further comprising: quantifying the drug resistance level of the cells with different drug resistance levels.

5. The identification method according to claim 1, further comprising: applying stimuli of different types and intensities during acquisition of the cell physical information,

   the stimuli comprise at least one of physical stimuli, chemical stimuli, and biological stimuli;
   optionally, said stimuli are a combination of one or more of physical stimuli, chemical stimuli, and biological stimuli;
   optionally, said physical stimuli, chemical stimuli, and biological stimuli comprise a combination of one or more stimulus forms from: drugs, mechanical force, stiffness, biochemicals, electric fields, flow fields, tropic guidance, and radiation.

6. The identification method according to claim 1, further comprising: comparing and analyzing results identified based on the cell physical information with results from biochemical and/or optical characterization, wherein the biochemical and/or optical characterization comprises at least one of protein staining, immunohistochemical staining imaging characterization, and single-cell sequencing.

7. The identification method according to claim 1, comprising performing characterization using a cell characterization system, wherein the cell characterization system comprises: a cell mechanical force detection device for acquiring cell mechanical force, wherein the cells have different drug resistance levels to a specific drug;

the cell mechanical force detection device comprises: a base, and a micropillar array composed of at least one micropillar that can deform under the cellular mechanical force of the cells disposed on the base,

the micropillars comprise a bottom end connected to the base, a side surface, a pillar body enclosed by the side surface, and a top end opposite the bottom end and away from the base, wherein the micropillars have comprise a light-reflective layer; the base and the pillar body of the micropillars comprises a light-transmissive portion, and the top end of the micropillars comprises a light-reflective layer.

8. The identification method according to claim 7, wherein the cell characterization system further comprises: an optical signal generation device and an optical signal detection device,

the optical signal generation device has a light source, and the light emitted from the light source illuminates the light-reflective layer via an incident light path;

the optical signal detection device is used to detect the light reflected from the light-reflective layer, wherein the light reflected from the light-reflective layer enters the optical signal detection device by a reflected light path;

the light intensity acquired by the optical signal detection device is linearly correlated with the cell mechanical force within a certain range.

9. The identification method according to claim 8, wherein the cell characterization system further comprises: an optical signal analysis device, which transforms the optical signal analysis results into visual information or data;

the optical signal analysis device is used to calculate the cell mechanical force of each micropillar based on attenuation of the reflected light intensity, and to determine the type, state, and behavior of the detected cells according to a preset model.

10. A cell identification and classification system based on drug sensitivity, comprising: an information acquisition unit and a pre-processing unit,

the information acquisition unit is for acquiring cell physical information, wherein the cell physical information comprises cell mechanical force and/or cell stiffness;

the pre-processing unit is for pre-processing the cell physical information to form structured cell information.

11. The cell identification and classification system according to claim 10, wherein the cell mechanical force comprises at least one of magnitude, direction, distribution, and frequency of the cell mechanical force or comprises at least one of magnitude, direction, distribution, and frequency of the cell mechanical force and a variation over time thereof;

the cell stiffness comprises the magnitude of cell stiffness at specific locations within the cell, spatial distribution of different layers at specific locations within the cell, and a variation over time thereof; the cell physical information is obtained before, during, and/or after the action of a specific drug.

12. The cell identification and classification system according to claim 10, wherein the cell physical information comprises at least one of cell number, cell mechanical force, cell stiffness, and cell morphology.

13. The cell identification and classification system according to claim 10, wherein the cell identification and classification system further comprises a learning unit and a recognition unit,

the learning unit is for using the structured cell information as input data to establish a cell feature model using supervised, unsupervised, or semi-supervised machine learning;

the recognition unit is for applying the cell feature model to the classification or clustering of cells based on drug sensitivity, to achieve the recognition of cell types based on drug sensitivity.

14. A method for acquiring cell physical information based on drug sensitivity, wherein the cell physical information comprises cell mechanical force and/or cell stiffness, comprising the following steps:

obtaining a number of original cells and acquiring the cell physical information of each original cell;

performing specific drug screening on the original cells to obtain cells with different drug resistance level;

acquiring the cell physical information of the cells with different drug resistance levels;

comparing the cell mechanical force information of the cells with different drug resistance levels with the cell mechanical force information of the corresponding original cells, wherein cells with a reduction in cell mechanical force less than a first preset threshold during and/or after drug treatment are drug-resistant cells, and cells with a reduction greater than or equal to a second preset threshold are non-drug-resistant cells; or comparing the cell stiffness of the cells with different drug resistance levels with the cell stiffness of the corresponding original cells, wherein cells with a reduction in cell stiffness less than a third preset threshold during and/or after drug treatment are drug-resistant cells, and cells with a reduction greater than or equal to a fourth preset threshold are non-drug-resistant cells; acquiring the cell physical information of the drug-resistant and non-drug-resistant cells for the specific drug.

15. A method for identification and classification of cells based on drug sensitivity, **characterized in that** the identification and classification are performed by acquiring the cell physical information of the cells; wherein the cell physical information comprises cell mechanical force information;

the cell mechanical force information comprises at least one of cell mechanical force information acquired before the action of external factors, during the action of external factors, and after the action of external factors; classifying cell types according to different drug resistance levels; the cell types include: normal cells and pathological cells, activated cells and non-activated cells; the cell mechanical force of drug-resistant cells with EGFR mutations is large and uniformly dispersed throughout the entire cell, while the cell mechanical force of non-drug-resistant cells is small and concentrated in the peripheral regions of the cell.

16. The method for identification and classification of cells based on drug sensitivity according to claim 15, comprising the following steps:

acquiring cell physical information of cells based on drug sensitivity, wherein the cell physical information includes cell mechanical force information and cell stiffness information at a certain point in the cell, the cell mechanical force information comprises the magnitude, direction, frequency, distribution, and dynamic change over time of the cell mechanical force at specific locations of the cell or multicellular aggregate, and the cell stiffness information comprises a magnitude of stiffness and spatial distribution of different layers at specific locations within the cell, , and a variation over time thereof; pre-processing the cell physical information to form structured cell information; the structured cell information comprises the number of cells, the number of cell features, and the feature information of each cell feature; using the structured cell information as input data to establish a cell feature model using supervised, unsupervised, or semi-supervised machine learning, and applying the cell feature model to the classification or clustering of cells based on drug sensitivity of unknown type or unknown state.

17. An application of the identification method according to any one of claims 1 to 9, the cells identification and classification system based on drug sensitivity as claimed in claim 10, the method for acquiring cell mechanical force of cells and/or multicellular aggregates based on drug sensitivity as claimed in claim 14, and the method for identification and classification of cells based on drug sensitivity as claimed in claim 15, wherein the application comprises: precision drug screening, screening of drug-resistant cells, and drug discovery.

18. The application according to claim 17, comprising:

classifying cells and/or multicellular aggregates by acquiring the cell mechanical force of two or more cells before, during, and after drug action, to predict the effectiveness of the drug and achieve precision screening of therapeutic drugs; or classifying a single cell's response to different drugs by acquiring its state before, during, and after the action of different drugs, to screen for the most effective drug or drug combination and achieve precision screening of therapeutic drugs

EP 4 692 300 A1

Figure 1

Figure 2

Figure 3

33

Figure 4

Figure 5

Figure 6

Figure 7

Figure 8

Figure 9

Figure 10

Figure 11

Figure 12

Figure 13

Figure 14

Figure 15

Figure 16

Figure 17

Figure 18

Figure 19

Figure 20

Figure 21

Figure 22

Figure 23

Figure 24

## INTERNATIONAL SEARCH REPORT

| International application No. |
| --- |
| **PCT/CN2024/083361** |

**A. CLASSIFICATION OF SUBJECT MATTER**

C12M 1/34(2006.01)i; C12M 1/00(2006.01)i; C12Q 1/02(2006.01)i; G06V20/69(2022.01)i

According to International Patent Classification (IPC) or to both national classification and IPC

**B. FIELDS SEARCHED**

Minimum documentation searched (classification system followed by classification symbols)

IPC:C12M,C12Q,G06V

Documentation searched other than minimum documentation to the extent that such documents are included in the fields searched

Electronic data base consulted during the international search (name of data base and, where practicable, search terms used)

CJFD, CNTXT, ENTXT, ENTXTC, PUBMED, SpringerLink, ISI Web of Knowledge, Elsevier Science Direct, Wiley InterScience, Nature, Science, CNKI, 万方, WANFANG: 细胞机械力, 细胞牵引力, 细胞力, 微柱, 反射, 药敏, 药效, 耐药, mechanical force, micro-column, array, drug effect.

**C. DOCUMENTS CONSIDERED TO BE RELEVANT**

| Category* | Citation of document, with indication, where appropriate, of the relevant passages | Relevant to claim No. |
| --- | --- | --- |
| PX | WO 2023046166 A1 (RUIXIN (FUZHOU) TECHNOLOGY CO., LTD.) 30 March 2023 (2023-03-30) claims 1-21, and description, paragraphs 52 and 219-225 | 1-18 |
| X | CN 115394456 A (SHENYANG INSTITUTE OF AUTOMATION, CHINESE ACADEMY OF SCIENCES) 25 November 2022 (2022-11-25) claims 1-10, and description, paragraphs 2 and 50 | 1-6, 10-18 |
| Y | CN 115394456 A (SHENYANG INSTITUTE OF AUTOMATION, CHINESE ACADEMY OF SCIENCES) 25 November 2022 (2022-11-25) claims 1-10, and description, paragraphs 2 and 50 | 7-9, 17-18 |
| Y | US 2015300953 A1 (THE UNIVERSITY OF NORTH CAROLINA AT CHAPEL HILL) 22 October 2015 (2015-10-22) claims 1-83, description, paragraphs 16-63, and figure 1 | 7-9, 17-18 |
| Y | CN 106338500 A (BEIJING INSTITUTE OF NANOENERGY AND NANOSYSTEMS) 18 January 2017 (2017-01-18) claims 1-14 | 7-9, 17-18 |

☑ Further documents are listed in the continuation of Box C. ☑ See patent family annex.

| | | | |
| --- | --- | --- | --- |
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
| --- | --- |
| **22 June 2024** | **03 July 2024** |

| Name and mailing address of the ISA/CN | Authorized officer |
| --- | --- |
| **China National Intellectual Property Administration (ISA/CN)** **China No. 6, Xitucheng Road, Jimenqiao, Haidian District, Beijing 100088** | |
| | Telephone No. |

Form PCT/ISA/210 (second sheet) (July 2022)

## INTERNATIONAL SEARCH REPORT

| International application No. |
| --- |
| **PCT/CN2024/083361** |

### C.     DOCUMENTS CONSIDERED TO BE RELEVANT

| Category* | Citation of document, with indication, where appropriate, of the relevant passages | Relevant to claim No. |
| --- | --- | --- |
| A | US 2019317050 A1 (HUNAN AGRICULTURAL UNIVERSITY) 17 October 2019 (2019-10-17)<br>     description, paragraphs 4 and 21-32 | 1-18 |
| A | US 2018372635 A1 (THE REGENTS OF THE UNIVERSITY OF CALIFORNIA) 27 December 2018 (2018-12-27)<br>     claims 1-51 | 1-18 |
| A | US 2020116696 A1 (THE REGENTS OF UNIVERSITY OF CALIFORNIA) 16 April 2020 (2020-04-16)<br>     claims 1-79, and description, paragraphs 115-117 | 1-18 |

Form PCT/ISA/210 (second sheet) (July 2022)

## INTERNATIONAL SEARCH REPORT
### Information on patent family members

International application No.

**PCT/CN2024/083361**

| Patent document cited in search report | | | Publication date (day/month/year) | Patent family member(s) | | | Publication date (day/month/year) |
|---|---|---|---|---|---|---|---|
| WO | 2023046166 | A1 | 30 March 2023 | None | | | |
| CN | 115394456 | A | 25 November 2022 | None | | | |
| US | 2015300953 | A1 | 22 October 2015 | US | 9952149 | B2 | 24 April 2018 |
| | | | | WO | 2014085804 | A1 | 05 June 2014 |
| | | | | EP | 2926115 | A1 | 07 October 2015 |
| | | | | EP | 2926115 | A4 | 06 July 2016 |
| | | | | EP | 2926115 | B1 | 26 May 2021 |
| CN | 106338500 | A | 18 January 2017 | WO | 2017008699 | A1 | 19 January 2017 |
| US | 2019317050 | A1 | 17 October 2019 | JP | 2019530442 | A | 24 October 2019 |
| | | | | JP | 6851651 | B2 | 31 March 2021 |
| | | | | EP | 3505925 | A1 | 03 July 2019 |
| | | | | EP | 3505925 | A4 | 08 April 2020 |
| | | | | EP | 3505925 | B1 | 28 June 2023 |
| | | | | WO | 2018041060 | A1 | 08 March 2018 |
| | | | | US | 11029285 | B2 | 08 June 2021 |
| | | | | AU | 2017319889 | A1 | 18 April 2019 |
| | | | | AU | 2017319889 | B2 | 06 January 2022 |
| US | 2018372635 | A1 | 27 December 2018 | WO | 2017015063 | A1 | 26 January 2017 |
| | | | | US | 10712271 | B2 | 14 July 2020 |
| US | 2020116696 | A1 | 16 April 2020 | US | 11782046 | B2 | 10 October 2023 |
| | | | | WO | 2019010234 | A1 | 10 January 2019 |

Form PCT/ISA/210 (patent family annex) (July 2022)